**Europäisches Patentamt**

**European Patent Office**

**Office européen des brevets**

(19)

(11) **EP 0 791 651 A1**

(12) **EUROPÄISCHE PATENTANMELDUNG**

(43) Veröffentlichungstag:
27.08.1997 Patentblatt 1997/35

(21) Anmeldenummer: 97100938.6

(22) Anmeldetag: 22.01.1997

(51) Int. Cl.$^6$: **C12N 13/00**, C12P 23/00,
C12P 1/04, A01G 7/04
// (C12P1/04, C12R1:01),
(C12P23/00, C12R1:01)

(84) Benannte Vertragsstaaten:
AT BE CH DE DK ES FI FR GB GR IE IT LI LU MC NL PT SE

(30) Priorität: 31.01.1996 CH 245/96
31.01.1996 CH 246/96

(71) Anmelder: IPR-Institute for Pharmaceutical Research Riehen A G
4125 Riehen (CH)

(72) Erfinder: Ebner, Guido, Dr.
5074 Eiken (CH)

(74) Vertreter: Goth, Helmut, Dr.
IPR-Institute for Pharmaceutical Research AG,
Lörracherstrasse 50
4125 Riehen (CH)

(54) **Method zur Behandelung von biologischem Material**

(57) Die vorliegende Erfindung beschreibt ein neuartiges Verfahren, welches, basierend auf einer Modifikation chemisch/physikalischer Prozessabläufe in einfachen sowie in komplexen Systemen zu wünschenswerten und nützlichen Veränderungen von diesen Systemen inhärenten Eigenschaften führt.

Ein wesentlicher Aspekt dieser Erfindung betrifft die Modifikation von chemisch/physikalischen Prozessabläufen in komplexen biologischen Systemen, die sich in einer wünschenswerten und nützlichen Veränderung bestimmter Eigenschaften der diese Systeme beinhaltenden Organismen manifestiert, wie beispielsweise einer Veränderung der Genexpressionsmuster, der Morphologie, der Entwicklungs- und Wachstumseffizienz, der Stressanfälligkeit u.a.m.

**Beschreibung**

Die vorliegende Erfindung beschreibt ein neuartiges Verfahren, welches, basierend auf einer Modifikation chemisch/physikalischer Prozessabläufe, in einfachen sowie in komplexen Systemen zu wünschenswerten und nützlichen Veränderungen von diesen Systemen inhärenten Eigenschaffen führt.

Ein wesentlicher Aspekt dieser Erfindung betrifft die Modifikation von chemisch/physikalischen Prozessabläufen in komplexen biologischen Systemen, die sich in einer wünschenswerten und nützlichen Veränderung bestimmter Eigenschaften der diese Systeme beinhaltenden Organismen manifestiert, wie beispielsweise einer Veränderung der Genexpressionsmuster, der Morphologie, der Entwicklungs- und Wachstumseffizienz, der Stressanfälligkeit u.a.m.

Ebenso umfasst von der vorliegenden Erfindung sind daher Organismen, wie zum Beispiel Mikroorganismen, Pilze, Pflanzen, wirbellose Tiere und Wirbeltiere aus den Klassen der Amphibien, Reptilien, Vögel und Säuger, die aufgrund des erfindungsgemässen Verfahrens bestimmte wünschenswerte und nützliche Veränderungen aufweisen, wie zum Beispiel eine erhöhte Entwicklungs- und Wachstumseffizienz, eine veränderte Genexpression, eine veränderte Morphologie, eine erhöhte Stressresistenz, eine veränderte Populationsdynamik u.a.m.

Das Interesse bei der Untersuchung von Parametern, die eine mittelbare oder unmittelbare Wirkung auf chemisch/physikalische Prozessabläufe ausüben, konzentrierte sich bisher fast ausschliesslich auf den Einfluss von Temperatur, Druck und elektromagnetischer Strahlung. Weit weniger Beachtung fand dagegen beispielsweise die Untersuchung über eventuelle Wechselwirkungen zwischen einfachen und komplexen Systemen, insbesondere zwischen komplexen biologischen Systemen und physikalischen Feldern.

Erst in jüngerer Zeit häufen sich die Berichte über mögliche Auswirkungen von Gravitation und magnetischen Feldern auf biologische Systeme. So konnten beispielsweise Goodman und Henderson (Bioelectromagnetics, 1986, 7, 23-29) Anzeichen dafür finden, dass ein Zusammenhang zwischen elektromagnetischen Feldern und der Transkriptionsrate in biologischem Material besteht, die von dem angelegten EM-Feld positiv beeinflusst wird, im Sinne einer Transkriptionssteigerung.

Die Möglichkeit, dass auch statische Elektrofelder einen Einfluss auf chemisch/physikalische Prozessabläufe in einfachen oder aber in komplexen Systemen, insbesondere auch in komplexen biologischen Systemen haben könnten, wurde dagegen bisher offenbar von vornherein ausgeschlossen. Entsprechend existieren bisher auch keine Berichte über eine mögliche Wirkung statischer Elektrofelder auf besagte Systeme. Dies mag in erster Linie dadurch begründet erscheinen, dass man gemäss der bisher gängigen Lehrmeinung davon ausging, dass ein statisches Elektrofeld in einem mit Ladungsträgern gefüllten Medium durch die spontane Ausbildung einer elektrischen Doppelschicht abgeschirmt wird und damit in seiner Wirkung inert bleibt.

Eine einzige Ausnahme bildet ein in der US Patentschrift Nr. 5,048,458 beschriebenes verbessertes Fischzuchtverfahren. Aufgrund dieses Standes der Technik konnte jedoch gemäss der gängigen Lehrmeinung nicht erwartet werden, dass dieses Verfahren zu einer generellen Methode ausgestaltet werden kann.

Diese Lehrmeinung basiert im wesentlichen auf der von C. Gouy und D.L. Chapman aufgestellten Beziehung, nach der die effektive Dicke einer diffusen Doppelschicht für einen Elektrolyten

$$d = \frac{1}{F} \cdot \sqrt{\frac{1000\varepsilon RT}{4\pi\Sigma c_i z_i^2}}$$

beträgt, worin d = die Dicke der Doppelschicht, F = die Faradayische Konstante, $\varepsilon$ = die Dielektrizitätskonstante, R = die universelle Gaskonstante, T = die absolute Temperatur und i = die Ionenarten der Konzentrationen $c_i$ und der Wertigkeiten $z_i$ bedeuten.

Dieses Vorurteil konnte jetzt im Rahmen der vorliegenden Erfindung durch Anwendung einfacher Verfahrensmassnahmen überraschenderweise überwunden werden.

Entgegen den zuvor näher charakterisierten Vorgaben der bisher gängigen Lehrmeinung ist es im Rahmen dieser Erfindung jetzt erstmals gelungen ein generelles Verfahren zu entwickeln, mit dessen Hilfe es möglich wird, basierend auf einer Modifikation chemisch/physikalischer Prozessabläufe, durch die Einwirkung eines statischen Elektrofeldes sowohl in einfachen als auch in komplexen Systemen, insbesondere in komplexen biologischen Systemen, wünschenswerte und nützliche Veränderungen zu induzieren. Dies kann am einfachsten dadurch erreicht werden, dass man besagte Systeme in ein statisches Elektrofeld einbringt, sodass die chemisch/physikalischen Prozesse, die beeinflusst werden sollen, unter dem Einfluss eines definierten elektrostatischen Feldes unter kontrollierbaren Bedingungen ablaufen.

Gegenstand der vorliegenden Erfindung ist somit ein Verfahren, welches basierend auf einer Modifikation chemisch/physikalischer Prozessabläufe in einfachen sowie in komplexen Systemen zu wünschenswerten nützlichen Veränderungen in diesen Systemen inhärenten Eigenschaffen führt und das sich dadurch kennzeichnet, dass man

a) besagte Systeme in ein statisches Elektrofeld einbringt, sodass die chemisch/physikalischen Prozesse, die beeinflusst werden sollen, unter dem Einfluss eines definierten elektrostatischen Feldes unter kontrollierbaren Bedingungen ablaufen, ohne dadurch aber die chemische Identität des Systems selbst zu verändern und

b) besagte Systeme dort für einen Zeitraum belässt, der für eine stabile Ausbildung der gewünschten Modifikation notwendig ist.

Die einfachen und komplexen Systeme, die im Rahmen der vorliegenden Erfindung in wünschenswerter und nützlicher Weise modifiziert werden, können umfassen in erster Linie physikalisch/chemische Systeme, wie z. B. chromatographische Systeme, Membranpermeabilitäten, Viskositäten, Löslichkeiten, Kinetik chemischer Reaktionen, Ionenaktivitäten, Kristallisationen, Präzipitationen, Konformationen von Molekülen oder Dielektrizitätskonstanten. Besonders bevorzugt sind komplexe biologische Systeme sowie insbesondere das diese Systeme beinhaltende biologische Material selbst.

Besonders bevorzugt im Rahmen dieser Erfindung ist daher die Anwendung dieses erfindungsgemässen Verfahrens auf komplexe biologische Systeme, bzw. auf die diese Systeme beinhaltenden Organismen, die zu einer Vielzahl unerwarteter Erscheinungen führt, die in vielfältiger Weise genutzt werden können, wie aus der folgenden detaillierten Beschreibung hervorgeht.

So führt die Anwendung des erfindungsgemässen Verfahrens auf komplexe biologische Systeme als Bestandteil eines intakten Organismus beispielsweise zu einer Veränderung der Entwicklungs- und Wachstumseffizienz, der Morphogenese, der Genexpressionsmuster, der Stressanfälligkeit, der Populationszusammensetzung u.a.m.

Ein wesentlicher Aspekt dieser Erfindung betrifft daher ein Verfahren zur Steigerung der Effizienz von Entwicklung und Wachstum von biologischem Material. Besonders bevorzugt ist ein Verfahren zur Steigerung der Effizienz von Entwicklung und Wachstum bei Pflanzen, wirbellosen Tieren und Wirbeltieren aus den Klassen der Amphibien, Reptilien, Vögel und Säuger.

Ebenso umfasst von der vorliegenden Erfindung ist das aus der Anwendung des erfindungsgemässen Verfahrens resultierende biologische Material selbst, insbesondere aber die aus der Anwendung des erfindungsgemässen Verfahrens resultierenden Pflanzen, wirbellosen Tieren und Wirbeltieren aus den Klassen der Amphibien, Reptilien, Vögel und Säuger, die eine erhöhte Entwicklungs- und Wachstumseffizienz aufweisen sowie die auf sexuellem und asexuellem Wege erzeugten Nachkommen der genannten Lebewesen, die noch diese neue und charakteristische Eigenschatten des Ausgangsmaterials besitzen.

Unter einer erhöhten Entwicklungs- und Wachstumseffizienz von Pflanzen, wirbellosen Tieren und Wirbeltieren aus den Klassen der Amphibien, Reptilien, Vögel und Säuger ist im Rahmen dieser Erfindung definitionsgemäss zum Beispiel eine Steigerung der Keimungsrate und der Keimungs- und Wachstumsgeschwindigkeit bei Pflanzen sowie eine Steigerung der Schlupf- bzw. Geburtenraten bei Tieren zu verstehen.

Weiterhin betrifft die vorliegende Erfindung ein Verfahren zur Beeinflussung der Morphogenese von biologischem Material, insbesondere aber ein Verfahren zur Beeinflussung der Morphogenese von Pflanzen.

Die vorliegende Erfindung umfasst somit auch biologisches Material sowie insbesondere Pflanzen, das aufgrund einer geänderten Morphogenese typische Veränderungen in seiner Morphologie erfahren hat. Für Pflanzen betreffen diese morphologischen Modifikationen in erster Linie die Form und Ausgestaltung von Blättern und Stengel, die Wuchsform sowie den Gesamthabitus der Pflanze.

Ebenso umfasst von der vorliegenden Erfindung ist ein Verfahren zur Änderung der Genexpressionsmuster von biologischem Material, insbesondere aber ein Verfahren zur Änderung der Genexpressionsmuster bei Pflanzen sowie das aus der Anwendung des erfindungsgemässen Verfahrens resultierende Material selbst und dessen Nachkommen.

Ein weiterer Gegenstand der vorliegenden Erfindung betrifft ein Verfahren, welches die spezifischen Stressreaktionen von biologischem Material in einer wünschenswerten und nützlichen Weise modifiziert. Diese gewünschte Modifikation der Stressreaktion kann auf sehr unterschiedliche Weise erfolgen, abhängig von dem verwendeten biologischen Material und der für dieses Material jeweils typischen Reaktion auf bestimmte Stresssituationen. So ist es beispielsweise möglich, die Stressanfälligkeit bestimmter Organismen durch Anwendung des erfindungsgemässen Verfahrens zu verringern und die Organismen somit unter Bedingungen zu kultivieren, die normalerweise kritisch sind für eine normale und geregelte Entwicklung besagter Organismen.

Ebenso umfasst von der vorliegenden Erfindung sind daher Organismen, deren spezifische Reaktionen auf bestimmte Stressparameter durch die Anwendung des erfindungsgemässen Verfahrens in einer gewünschten und nützlichen Art und Weise modifiziert sind, insbesondere solche, die eine erhöhte Resistenz gegenüber Stress aufweisen.

Insbesondere umfasst von der vorliegenden Erfindung sind Pflanzen, die aufgrund der Anwendung des erfindungsgemässen Verfahrens in der Lage sind, auch ausserhalb der für sie typischen Wachstumsperiode zu wachsen und sich zu vermehren sowie die auf sexuellem und asexuellem Wege erzeugten Nachkommen dieser Pflanzen, die noch diese neue und charakteristische Eigenschaften der Mutterpflanze aufweisen.

Ein weiterer Gegenstand der vorliegenden Erfindung betrifft ein Verfahren, welches eine direkte Induktion des Sekundärstoffwechsels bei Organismen ermöglicht, die über einen solchen verfügen, und das auf diese Weise die Produktion von Sekundärmetaboliten ohne die Anwendung aufwendiger Verfahrensmassnahmen möglich macht.

Ebenso umfasst von der vorliegenden Erfindung sind die auf diese Weise hergestellten Sekundärmetaboliten produzierenden Organismen sowie die von diesen produzierten Verbindungen.

Gegenstand der vorliegenden Erfindung ist somit ein allgemein anwendbares Verfahren, welches basierend auf einer Modifikation chemisch/physikalischer Prozessabläufe in einfachen sowie in komplexen Systemen zu wünschenswerten und nützlichen Veränderungen von diesen Systemen inhärenten Eigenschaften führt und das durch die folgenden Verfahrensmassnahmen gekennzeichnet ist:

a) Einbringen von einfachen oder komplexen Systemen, in denen die spezifischen chemisch/physikalischen Prozessabläufe stattfinden, welche mit Hilfe des erfindungsgemässen Verfahrens modifiziert werden sollen, in ein statisches Elektrofeld,
b) Einstellen der für die jeweils gewünschte Modifikation der im statischen Elektrofeld befindlichen Systeme geeigneten Feldparameter und
c) Belassen der besagten Systeme im statischen Elektrofeld für einen Zeitraum, der für das Auftreten und gegebenenfalls für die Erhaltung der gewünschten Modifikation notwendig ist.

Besonders bevorzugt im Rahmen dieser Erfindung ist ein Verfahren, welches basierend auf einer Modifikation chemisch/physikalischer Prozessabläufe in einfachen sowie in komplexen biologischen Systemen zu wünschenswerten und nützlichen Veränderungen von diesen Systemen inhärenten Eigenschaften führt und das durch die folgenden Verfahrensmassnahmen gekennzeichnet ist:

a) Einbringen von biologischem Material in ein statisches Elektrofeld,
b) Einstellen der für die jeweils gewünschte Modifikation geeigneten Feldparameter und
c) Belassen des biologischen Materials im statischen Elektrofeld für einen Zeitraum, der für das Auftreten und gegebenenfalls für die Erhaltung der gewünschten Modifikation notwendig ist.

Im Rahmen der vorliegenden Erfindung wird das statische Elektrofeld vorzugsweise zwischen den Platten eines Kondensators aufgebaut. Die elektrische Feldstärke des statischen Elektrofeldes ist durch die folgende Beziehung gegeben:

$$E = \frac{U}{d'}$$

worin U die Spannungsdifferenz zwischen den Kondensatorplatten und d' der Plattenabstand bedeuten. Die Form der Kondensatorplatten ist jedoch für die Zwecke der vorliegenden Erfindung ohne Bedeutung und kann den experimentellen Bedürfnissen angepasst werden. So kann z.B. ein Rundkolben, in welchem ein Metallstab eingeführt und der aussen mit einem für Kondensatoren geeigneten Material umgeben ist, Verwendung finden.

Die Spannungsdifferenz U wird durch einen Hochspannungsgenerator erzeugt. Es können beliebige Hochspannungsgeneratoren im Rahmen dieser Erfindung verwendet werden; bevorzugt sind Hochspannungsgeneratoren, die auf dem Transformatorprinzip mit Gleichrichter beruhen. Andere geeignete Spannungsquellen sind z.B. Batterien, van der Graff-Generatoren und Elektrisiermaschinen. Die Spannung ist eine kontinuierliche oder pulsierende Gleichspannung.

Die im Rahmen dieser Erfindung bevorzugte Spannungsdifferenz beträgt zwischen 1,0 V (Volt) und $10^5$ V, abhängig von der Art des zu modifizierenden Systems. Für die Anwendung des erfindungsgemässen Verfahrens auf biologisches Material verwendet man vorzugsweise eine Spannungsdifferenz von 1 V bis 20'000 V, insbesondere von 100 V bis 10'000 V. Ganz besonders bevorzugt ist eine Spannungsdifferenz von 500 V bis 3'000 V.

Der Plattenabstand des Kondensators richtet sich nach den Dimensionen der Probengefässe und beträgt z.B. zwischen 10 A und 10 m, vorzugsweise aber zwischen 1 cm und 50 cm.

Die Feldstärke des statischen Feldes kann bei gegebenem Plattenabstand durch die Höhe der Spannung am Hochspannungsgenerator reguliert werden.

Im Rahmen der vorliegenden Erfindung betragen die verwendeten Feldstärkewerte zwischen 1 V/cm und 10'000 V/cm, vorzugsweise zwischen 50 V/cm und 5'000 V/cm. Der für die Anwendung des erfindungsgemässen Verfahrens auf biologisches Material bevorzugte Bereich für die Feldstärke liegt bei 50 V/cm und 5'000 V/cm. Ganz besonders bevorzugt ist eine Feldstärke zwischen 500 V/cm und 2'000 V/cm.

Besonders bevorzugt im Rahmen dieser Erfindung ist die Anwendung des erfindungsgemässen Verfahren zur Modifikation von biologischem Material.

Unter biologischem Material ist im Rahmen dieser Erfindung definitionsgemäss sowohl ein intakter lebender Organismus, wie beispielsweise ein Mikroorganismus, ein Pilz, eine Pflanze, ein wirbelloses Tier oder ein Wirbeltier aus den Klassen der Amphibien, Reptilien, Vögel oder Säugern, als auch lebensfähige Teile besagter Organismen zu verstehen, die für eine bestimmte Zeit in Kultur gehalten werden können und die gegebenenfalls in der Lage sind, in Kultur zu wachsen und sich dort auch zu vermehren. Unter dem Begriff Mikroorganismen sind erfindungsgemäss neben Bakterien und Viren in erster Linie ein- bis vielzellige Algen sowie ein- bis vielzellige Pilze zu verstehen. Lebensfähige Teile von Organismen umfassen z.B. Protoplasten, haploide, diploide sowie polyploide Zellen und Gewebe, isolierte Zellorganellen, wie Zellkerne, Mitochondrien oder Chloroplasten, Kalli, Zellaggregate, Organe, Keimzellen (z.B. Antheren), Zygoten sowie Embryonen, die in der Lage sind, in Kultur zu wachsen und sich dort gegebenenfalls zu vermehren. Auch humane Zellen sind in diesem Zusammenhang zu erwähnen. Handelt es sich bei besagten Organismen um pflanzliche Organismen, so sind unter lebensfähigen Teilen darüber hinaus auch Zellaggregate, Kali, Organe, Samen, Sporen, Pollen und andere pflanzenspezifische Strukturen zu verstehen.

Ganz besonders bevorzugt im Rahmen dieser Erfindung ist biologisches Material, das eine hohe Teilungsaktivität aufweist und/oder noch wenig differenziert ist, wie z.B. teilungsaktive Zellen, Geschlechtszellen während des Befruchtungsvorgangs, Keimzellen, embryonale Zellen und Gewebe, Embryonen, Zygoten, Keimlinge oder meristematische Zellen und Gewebe.

Alle diese beispielhaften Aufzählungen dienen nur der Verdeutlichung der vorliegenden Erfindung und schränken den Erfindungsgegenstand in keiner Weise ein.

Ein Hauptgegenstand der vorliegenden Erfindung betrifft die Anwendung des erfindungsgemässen Verfahrens zur phänotypischen Modifikation von Pflanzen, insbesondere ein Verfahren zur Steigerung von Keimungs- und Wachstumsgeschwindigkeit, der Biomassen- und Samenproduktion und zur Modifikation der pflanzlichen Morphologie aufgrund einer geänderten Morphogenese sowie gegebenenfalls zur Modifikation der Genexpressionsmuster der behandelten Pflanzen.

Ein weiterer Gegenstand dieser Erfindung betrifft die modifizierte Pflanze selbst und deren Nachkommen sowie sämtliche Mutanten und Varianten davon, soweit sie noch die entsprechenden Modifikationen aufweisen.

Angesichts des raschen Anstiegs der Weltbevölkerung und dem damit verbundenen höheren Nahrungsmittel- und Rohstoffbedarf gehört die Steigerung des Ertrags von Nutzpflanzen sowie die vermehrte Gewinnung von pflanzlichen Inhaltsstoffen, also den Fortschritt auf dem Gebiet der Nahrungs- und Heilmittel, zu den dringlichsten Aufgaben der biologischen bzw. biotechnologischen Forschung. In diesem Zusammenhang sind als wesentliche Aspekte beispielsweise zu nennen:

a) eine Erhöhung der Resistenz von Nutzpflanzen gegen ungünstige Bodenverhältnisse oder gegen Krankheiten und Schädlinge,
b) eine gesteigerte Resistenz gegen Pflanzenschutzmittel wie Insektizide, Herbizide, Fungizide und Bakterizide,
c) und insbesondere eine günstige Veränderung des Nährstoffgehalts oder des Ernteertrags von Pflanzen.

Solche wünschenswerte Effekte könnten allgemein durch Induktion oder vermehrte Bildung von Schutzstoffen, wertvollen Proteinen oder Toxinen sowie durch Eingriffe in die regulatorischen Kreisläufe des Pflanzenmetabolismus herbeigeführt werden. Dies kann beispielsweise durch Beeinflussung des pflanzlichen Erbgutes mit Hilfe traditioneller Züchtungsverfahren, wie sie in der Pflanzenzüchtung seit langem etabliert sind, erreicht werden.

Eine weitere Möglichkeit der gezielten Beeinflussung des pflanzlichen Erbgutes besteht in einer Übertragung von zuvor isolierten Genen, die für neue, nützliche und wünschenswerte Eigenschaften kodieren, in ganze Pflanzen oder Pflanzenzellen mit Hilfe von in jüngster Vergangenheit neu entwickelten Verfahren zur genetischen Modifikation von höheren Pflanzen.

Beispielhaft seien in diesem Zusammenhang die auf der Verwendung des Agrobacterium-Transformationssystems beruhenden Gentransfer-Verfahren erwähnt (Eckes, P. et al., Angew. Chem. Int. Eng., 1987, 26, 382-402).

Alle diese zuvor genannten Verfahren sind aber mit zum Teil gravierenden Nachteilen verbunden. So erwiesen sich beispielsweise die traditionellen Züchtungsverfahren in der Regel als sehr zeit- und kostenintensiv, so dass in diesem Bereich nur langsame und allmähliche Fortschritte zu erwarten sind. Auch die Anwendung von neuen Gentechnologien auf dem Agrarsektor zur Einschleusung fremden, genetischen Materials in Pflanzen und der damit verbundenen Erwartung neuer Pflanzen mit nützlichen wünschenswerten Eigenschaften, ist noch mit zahlreichen und zur Zeit noch weitgehend ungelösten Problemen verbunden. Dies trifft in erster Linie auf Pflanzen aus der Ordnung der Monocotyledonae zu, welche die Mehrzahl der aus agrarökonomischer Sicht bedeutendsten Kulturpflanzen beinhaltet. Von besonderem Interesse ist die Familie der Gramineae, die unsere wichtigsten Getreidepflanzen umfasst, wie z.B. Weizen, Gerste, Roggen, Hafer, Mais, Reis, Hirse u.a.m.

Es muss daher als eine vordringliche Aufgabe angesehen werden, Verfahren zu entwickeln, die eine schnelle, effiziente und kostengünstige Veränderung bestimmter, aus agrarökonomischer Sicht relevanter Eigenschaften von Pflanzen ermöglicht. Diese Aufgabe konnte jetzt im Rahmen dieser Erfindung überraschenderweise mit einfachen Massnahmen gelöst werden.

Gegenstand der vorliegenden Erfindung ist somit ein Verfahren zur phänotypischen Modifikation von Pflanzen, insbesondere von Kulturpflanzen sowie gegebenenfalls zur Modifikation der pflanzlichen Genexpressionsmuster.

Weiterhin betrifft diese Erfindung das mit Hilfe des erfindungsgemässen Verfahrens hergestellte pflanzliche Material selbst und dessen Nachkommen sowie sämtliche Mutanten und Varianten davon, soweit diese noch die entsprechenden Modifikationen aufweisen. Unter Nachkommen sind im Rahmen dieser Erfindung alle auf sexuellem oder asexuellem Wege hergestellten Abkömmlinge des modifizierten biologischen Materials zu verstehen, insbesondere auch solche, die mit Hilfe von Regenerationstechniken aus pflanzlichen Zellen oder Protoplasten regeneriert worden sind und noch die typischen Eigenschaften des Ausgangmaterials aufweisen. Unter Mutanten und Varianten sind im Rahmen dieser Erfindung sämtliche durch künstliche oder natürliche Mutagenese hergestellten Abkömmlinge des modifizierten biologischen Materials, insbesondere des erfindungsgemässen pflanzlichen Materials zu verstehen, die noch die typischen Eigenschaften des Ausgangsmaterials aufweisen.

Im einzelnen beschreibt diese Erfindung ein Verfahren zur Herstellung von Pflanzen mit gegenüber dem Ausgangsmaterial veränderten, nützlichen und wünschenswerten Eigenschaften, das sich dadurch kennzeichnet, dass man die Samen besagter Pflanzen in ein statisches Elektrofeld einbringt und diese dort zur Keimung gelangen lässt.

Das erfindungsgemässe Verfahren ist durch die folgenden Verfahrensmassnahmen charakterisiert:

a) Einbringen von pflanzlichem Material in ein statisches Elektrofeld,
b) Einstellen der für die jeweils gewünschte Modifikation geeigneten Feldparameter,
c) Belassen des pflanzlichen Materials im statischen Elektrofeld für einen Zeitraum, der für das Auftreten und gegebenenfalls für die Erhaltung der gewünschten Modifikation notwendig ist.

Bei besagtem pflanzlichem Material kann es sich definitionsgemäss sowohl um ganze Pflanzen als auch um lebensfähige Teile von Pflanzen handeln, die in Kultur gehalten werden können und die gegebenenfalls in der Lage sind, in Kultur zu wachsen und sich dort auch zu vermehren, wie z.B. pflanzliche Protoplasten, Zellen, isolierte Zellorganellen wie Zellkerne, Mitochondrien oder Chloroplasten, Kalli, Zellaggregate, Gewebe, Organe, Keimzellen, Zygoten oder Embryonen. Besonders bevorzugt im Rahmen dieser Erfindung ist pflanzliches Material, das eine hohe Teilungsaktivität aufweist und/oder noch wenig differenziert ist, wie z.B. teilungsaktive Zellen, Keimzellen, embryonale Zellen und Gewebe, Zygoten, Embryonen, Keimlinge, meristematische Zellen und Gewebe u.a. Ganz besonders bevorzugt für die Verwendung in dem erfindungsgemässen Verfahren sind pflanzliche Embryonen, insbesondere Embryonen, die in Form von Samen vorliegen, sowie einzellige Fortpflanzungseinheiten, die in Form von Sporen vorliegen. Darüber hinaus können aber auch beispielsweise Zellkulturen in dem erfindungsgemässen Verfahren eingesetzt werden, insbesondere Kulturen von Keimbahnzellen, wie z.B. Antheren-, Ähren- oder Mikrosporenkulturen.

Das erfindungsgemässe Verfahren ist somit in erster Linie dadurch gekennzeichnet, dass man

a) Samen oder Sporen in ein statisches Elektrofeld einbringt,
b) besagte Samen oder Sporen unter standardisierten Licht-, Temperatur- und Feuchtigkeitsverhältnissen im statischen Elektrofeld ankeimt und
c) die Keimlinge dem Einflussbereich des statischen Elektrofeldes entnimmt, in Erde überführt und wie normale Keimlinge unter Anwendung bekannter Kultivierungsverfahren weiterkultiviert.

Bei der Verwendung von Samen oder Sporen hängt die Inkubationsdauer in entscheidendem Masse von der Art des verwendeten Materials sowie der gewählten Feldstärkewerte des statischen Elektrofeldes ab. Bevorzugt ist eine Inkubation der pflanzlichen Samen oder Sporen über einen Zeitraum von 0,1 bis 360 Tagen, insbesondere aber von 2 bis 8 Tagen für Samen und 1 bis 180 Tagen für Sporen. Dabei werden die Samen bzw. Sporen in einem geeigneten Keimungsmedium, vorzugsweise aber in Wasser, in das Dielektrikum eines Kondensators eingebracht, dessen Platten zuvor mittels eines Hochspannungsgenerators aufgeladen werden.

Die bevorzugten Feldstärkewerte des sich im Innern des Kondensators aufbauenden statischen Elektrofeldes betragen zwischen 10 V/cm und 10'000 V/cm, vorzugsweise aber zwischen 50 V/cm und 5'000 V/cm. Ganz besonders bevorzugt im Rahmen dieser Erfindung sind Feldstärkewerte von 750 V/cm bis 1'500 V/cm.

Die Wirksamkeit der gewählten Feldstärke ist von verschiedenen Faktoren abhängig, so z.B. von der Art des gewählten Pflanzenmaterials sowie des verwendeten Keimungsmediums.

In einer spezifischen Ausführungsform der vorliegenden Erfindung werden die Samen der Versuchspflanzen über einen Zeitraum von 1 bis 7 Tagen in einem statischen Elektrofeld in Wasser angekeimt. Die Feldstärkewerte betragen dabei zwischen 750 V/cm und 1'500 V/cm. Bei der Verwendung von Sporen, wie z.B. Farnsporen, kann die Keimphase entsprechend länger dauern und mehrere Wochen bis Monate in Anspruch nehmen.

Die Keimung erfolgt vorzugsweise unter standardisierten Bedingungen, insbesondere unter standardisierten Licht- und Temperaturverhältnissen in einer Dunkelkammer, deren Innenraum von einer Pflanzenlampe erleuchtet wird, da die Keimung von Samen und Sporen vom Licht stark beeinflusst wird.

Anschliessend werden die gekeimten Samen bzw. Sporen in Erde überführt und wie normale Keimlinge unter Anwendung bekannter Kultivierungsverfahren weiterkultiviert.

Bei der Verwendung von Farnsporen muss vor der Überführung in Erde die Entwicklung des Protalliums abgewartet werden, das sich in der Regel nach 12 Monaten voll ausgebildet hat. Nach der Befruchtung der Protallien beginnt dann die Entwicklung der Farnpflänzchen.

Die durch Anwendung des erfindungsgemässen Verfahrens hervorgerufenen Effekte, die einzeln oder in Kombination auftreten können, umfassen beispielsweise:

a) Steigerung der Keimungsrate der im statischen Elektrofeld behandelten Samen bzw. Sporen.
b) Beschleunigtes Wachstum der aus dem behandelten Samen-/Sporenmaterial erhaltenen Pflanzen.
c) Steigerung der Biomasseproduktion der aus dem behandelten Samen-/Sporenmaterial erhaltenen Pflanzen, z.B. durch Ausbildung weiterer Fruchtstände.
d) Steigerung des Samenertrages der aus dem behandelten Samen-/Sporenmaterials erhaltenen Pflanzen.
e) Änderungen der Morphologie des entstehenden Phänotyps der aus dem behandelten Samen-/Sporenmaterial erhaltenen Pflanzen.
f) Geänderte Genexpressionsmuster der aus dem behandelten Samen-/Sporenmaterial erhaltenen Pflanzen.
g) Änderung von spezifischen Lebensgewohnheiten der aus dem behandelten Samen-/Sporenmaterial erhaltenen Pflanzen, z.B. Übergang von einer einjährigen zu einer mehrjährigen Lebensweise.

Diese Effekte lassen sich auch bei Pflanzenmaterial beobachten, das unter dem Einfluss eines statischen Elektrofeldes aus pflanzlichen Protoplasten, Zell-, Kallus-, Gewebe- oder Organkulturen regeneriert wird. Neben diesen morphologischen Modifikationen findet man darüber hinaus eine leicht erhöhte Regerationsrate im Vergleich zu den Kontrollen.

Die beobachteten Modifikationen können auch auf die Nachkommen besagten Pflanzenmaterials übertragen werden.

Die durch die Anwendung des erfindungsgemässen Verfahrens auftretenden phänotypischen Veränderungen an dem im statischen Elektrofeld behandelten Pflanzenmaterial können in vielfältiger Weise genutzt werden, z.B. bei der Entwicklung von verbessertem Saatgut:

a) Steigerung der Keimungsrate und der Wachstumsgeschwindigkeit kann beispielsweise ausgenutzt werden zur Entwicklung von Pflanzen, die in der Lage sind, auch bei ungenügenden Lichtverhältnissen auszukeimen und damit für eine frühere Aussaat in biologischen Randgebieten, die eine nur kurze Vegetationsperiode aufweisen, geeignet sind.
b) Pflanzen, die Eigenschaften von Vorfahren aufweisen, können für Einkreuzungen mit Nutzpflanzen verwendet werden, um damit degenerative Verluste auf der genetischen Ebene auszugleichen. Als Beispiel soll hier die Entwicklung von 'mehrjährigem' Saatgut von sonst einjährigem Weizen genannt werden.
c) Pflanzen, die normalerweise eine beschränkte Anzahl Fruchtstände aufweisen, können dazu gebracht werden, bedeutend mehr Fruchtstände zu erzeugen. Beispielsweise kann Mais dazu veranlasst werden, anstatt 1 bis 2 Kolben bis zu 10 Kolben zu entwickeln. Damit kann die Ertragsausbeute pro bebauter Flächeneinheit bedeutend gesteigert werden.

Das zuvor beschriebene erfindungsgemässe Verfahren ist auf alle Pflanzen anwendbar, insbesondere auch solche aus der systematischen Gruppe der Angiospermae und Gymnospermae. Unter den Gymnospermae sind die Pflanzen aus der Klasse Coniferae von besonderem Interesse. Unter den Angiospermae sind neben den Laubbäumen und Sträuchern vor allem Pflanzen der Familien Solanaceae, Cruciferae, Compositae, Liliaceae, Vitaceae, Chenopodiaceae, Rutaceae, Bromeliaceae, Rubiaceae, Theaceae, Musaceae, Gramineae oder Leguminosae und hier vor allem der Familie Papilionaceae von besonderem Interesse. Bevorzugt sind Vertreter der Familien Solanaceae, Cruciferae, Leguminosae und Gramineae. Besonders bevorzugt sind dabei Vertreter aus der Familie der Gramineae, wie z.B. Pflanzen, die grossflächig angebaut werden und hohe Ernteerträge liefern. Als Beispiele seien genannt: Mais, Reis, Weizen, Gerste, Roggen, Hafer, Hirse, Sorghum u.a.m., ohne aber dadurch den Gegenstand der vorliegenden Erfindung in irgendeiner Weise zu limitieren.

Als mögliche Zielkulturen für die Anwendung des erfindungsgemässen Verfahrens sind somit beispielsweise Pflanzen der Gattungen Allium, Avena, Hordeum, Oryzae, Passicum, Saccharum, Secale, Setaria, Sorghum, Triticum, Zea, Musa, Cocos, Phoenix und Elaeis zu nennen, ohne damit jedoch den Erfindungsgegenstand in irgendeiner Weise zu limitieren.

Das erfindungsgemässe Verfahren ist jedoch nicht auf die Anwendung bei Pflanzen beschränkt, sondern kann in genau analoger Weise auch zur Steigerung der Entwicklungs- und Wachstumseffizienz z.B. bei wirbellosen Tieren und Wirbeltieren aus den Klassen der Amphibien, Reptilien, Vögel und Säugern angewendet werden.

Ein weiterer Gegenstand der vorliegenden Erfindung betrifft die Anwendung des erfindungsgemässen Verfahrens zur Modifikation spezifischer Stressreaktionen von biologischem Material. Besonders bevorzugte Anwendungsbereiche betreffen die Erhöhung der Stressresistenz z.B. gegenüber bestimmten Umweltfaktoren, wie erhöhten Salzkonzentrationen im Kulturmedium, Limitierung essentieller Nährstoffe, Limitierung von Licht und/oder $O_2$-/$CO_2$-Versorgung u.a.m.

Eine der häufigsten Stressfaktoren in der belebten Umwelt betrifft den Mangelstress, wobei ein oder aber mehrere Faktoren gleichzeitig für einen gegebenen Organismus limitierend wirken können. Mangelstress tritt auf, sobald einer oder mehrere der für ein optimales Wachstum oder eine optimale Entwicklung notwendigen Faktoren, wie Licht, $O_2/CO_2$-Versorgung, Nährstoffangebot, Vitamine etc. suboptimale Werte erreicht. Dies führt dazu, dass der betreffende Organismus bestimmte, für eine optimale Entwicklung und ein optimales Wachstum notwendige Syntheseleistungen nicht mehr in vollem Umfang aufrecht erhalten kann, was zunächst zu einer Verlangsamung des Wachstums führt. Hält dieser Mangelstress über einen längeren Zeitraum an, so führt dies schliesslich zu einer Beeinträchtigung essentieller Lebensfunktionen, was schliesslich in der Regel zu einem frühzeitigen Einsetzen der Seneszenz und damit letztlich zum Absterben des betroffenen Organismus führt. Dies lässt sich beispielsweise bei Weizenkeimlingen beobachten, bei denen eine starke Reduktion in der Lichtzufuhr zu einer Bräunung der Keimblätter und schliesslich zum Absterben des Keimlings führt.

Die gleichen Beobachtungen lassen sich auch bei in Kultur gehaltenen Grünalgenkolonien machen, die bei einer Reduktion der Lichtintensität über einen längeren Zeitraum hinweg, ebenfalls mit einer Braunfärbung reagieren. Diese Braunfärbung resultiert aus dem oxidativen Abbau des Chlorophylls, das durch Neusynthese nicht mehr ersetzt werden kann.

Im Rahmen der vorliegenden Erfindung ist es jetzt überraschenderweise gelungen, die Stressresistenz von biologischem Material, insbesondere aber die Stressresistenz von Bakterien und Pflanzen zu erhöhen, indem man besagtes Material unter Anwendung des erfindungsgemässen Verfahrens in ein statisches Elektrofeld einbringt und dort für einen Zeitraum belässt, der für die Ausprägung und gegebenenfalls für die Erhaltung besagter Stressresistenz notwendig ist.

Die Feldparameter sind zu einem gewissen Grade abhängig von dem verwendeten biologischen Material. Sie liegen vorzugsweise bei 50 V/cm bis 5'000 V/cm, besonders bevorzugt sind Feldstärkeeinheiten von 500 V/cm bis 1'500 V/cm.

Durch die Anwendung des erfindungsgemässen Verfahrens ist es beispielsweise möglich, das Einsetzen der Seneszenz als Reaktion auf einen Mangelstress bei Pflanzen zu verzögern.

Weizenkeimlinge sowie Algenkulturen, die bei suboptimalen Beleuchtungsstärken in Gegenwart eines statischen Elektrofeldes wachsen, zeigen beispielsweise gegenüber den Kontrollpflanzen ohne Elektrofeld deutlich gesteigerte Überlebensraten.

Eine weitere Anwendungsmöglichkeit des erfindungsgemässen Verfahrens betrifft die Steigerung der Toleranz von biologischem Material, insbesondere aber von Bakterien, gegenüber erhöhten Salzkonzentrationen im umgebenden Medium.

Nicht nur ein Mangel, sondern auch ein Überangebot an bestimmten kritischen Faktoren kann zur Auslösung von Stressreaktionen führen. Erhöhte Salzkonzentration im Nährmedium beispielsweise führt zu einer Erhöhung des osmotischen Wertes im Medium und infolgedessen zu einem Flüssigkeitsverlust der darin befindlichen Zellen infolge einer einsetzenden Osmose und damit zum Schrumpfen der Zellen. Der Flüssigkeitsverlust kann in der Regel durch ausgleichende Massnahmen der betroffenen Organismen in gewissem Rahmen kompensiert werden. Ist jedoch ein kritischer Schwellenwert überschritten, so führt dies zum Absterben der betroffenen Zellen.

Im Rahmen der vorliegenden Erfindung ist es jetzt überraschenderweise gelungen, die Salztoleranz von lebenden Zellen zu erhöhen, indem man besagte Zellen in Gegenwart eines statistischen Elektrofeldes schrittweise an hohe Salzkonzentrationen adaptiert.

Die für eine erfolgreiche Adaptation geeigneten Feldparameter sind zu einem gewissen Grade vom verwendeten biologischen Material abhängig. Bevorzugt sind Feldstärkewerte zwischen 500 V/cm und 2'000 V/cm, insbesondere aber zwischen 750 V/cm und 1'500 V/cm.

Die Wirksamkeit des erfindungsgemässen Verfahrens lässt sich beispielsweise an ubiquitären Bakterien demonstrieren, die schrittweise an hohe Salzkonzentrationen im umgebenden Medium adaptiert werden können, ohne dadurch jedoch den Umfang der Erfindung in irgendeiner Weise zu limitieren. Auf diese Weise ist es ohne weiteres möglich, durch die Anwendung des erfindungsgemässen Verfahrens ubiquitäre Bakterien in einem ersten Adaptationsschritt an Salzkonzentrationen bis zu 14 % zu gewöhnen. In einem zweiten Adaptationsschritt können im statischen Elektrofeld selbst Anpassungen an Salzkonzentrationen bis zu 28 % erreicht werden. Demgegenüber lassen sich bei Kontrollkulturen, die ausserhalb eines statischen Elektrofeldes an hohe Salzkonzentrationen adaptiert werden, im gleichen Zeitraum nur Anpassungen bis 3,5 % erreichen.

Die mit Hilfe des erfindungsgemässen Verfahrens nunmehr vorhandene Möglichkeit zur schnellen Adaptation ubiquitär vorkommender Bakterien an hohe Salzkonzentrationen ist von grossem technischem Interesse. So stellen beispielsweise rasch wechselnde Salzkonzentrationen in der biologischen Stufe von Kläranlagen ein seit langem bekanntes und bisher weitgehend ungelöstes Problem dar, das jetzt mit Hilfe des erfindungsgemässen Erfahrens auf einfache und effiziente Weise gelöst werden kann.

Ein weiteres Phänomen, das in unmittelbarem Zusammenhang mit einem Mangelstress steht und das damit einer Modifikation durch das erfindungsgemässe Verfahren zugänglich ist, betrifft die Regulation des Sekundärstoffwechsels von Bakterien und Pilzen.

Es ist bekannt, dass viele Bakterien und Pilze beim Auftreten von Mangelzuständen, wie beispielsweise einer Nährstofflimitierung im Medium oder einer $O_2$-Limitierung u.a. ihr Wachstum einstellen oder aber zumindest stark reduzieren und ihren Stoffwechsel auf die Produktion von sogenannten Sekundärmetaboliten umstellen. Bei besagten Sekundärmetaboliten handelt es sich vielfach um Verbindungen mit hoher pharmakologischer Wirksamkeit, wie z.B. um antibiotisch, antifungizid oder zytotoxisch wirksame Substanzen, die von grossem kommerziellem Interesse sind.

Die Herstellung dieser Verbindungen in ausreichender Menge im Rahmen von grosstechnischen Fermentationsprozessen ist oft mit enormen Schwierigkeiten verbunden und daher vielfach nur unter grossem Kostenaufwand realisierbar. Das Hauptproblem betrifft dabei die Steuerung der Prozessparameter, die in einer Weise erfolgen muss, dass der Produzentenstamm optimale Bedingungen für die Herstellung des gewünschten Produktes vorfindet.

Ein in der Praxis relevantes Beispiel für die Anwendung des erfindungsgemässen Verfahrens betrifft die Züchtung Rhodopsin- und β-Carotin-bildender Halobakterien. Bakteriorhodopsin geniesst weltweites Interesse als potentielle $K^+/Na^+$-Pumpe einerseits und käme andererseits als Mittel der Wahl für die Entwicklung biologischer Chips in Betracht. β-Carotin ist als Naturstoff und Ausgangmaterial für Vitamin A in Biologie und Medizin gleichermassen von Interesse. Die Analyse von Halobakterien hat neben dem Rhodopsin und β-Carotin ebenfalls ein Vorkommen an Vitamin-A-Säure, bevorzugt 13-cis-Vitamin-A-Säure, und Benzaldehyd ergeben. Klinische Vorversuche an Psoriasis-Patienten haben ausserdem gezeigt, dass erkrankte Hautbezirke, die mit einem Lysat von Halobacterium halobium behandelt werden, vollständig abheilen.

Es sind deshalb umfassende Anstrengungen unternommen worden, die Rhodopsin- und β-Carotin-bildende Form von Halobakterium halobium in grösserem Massstab zu züchten. Dazu wird normalerweise das Bakterium in seiner natürlich vorkommenden Variante kultiviert. Dazu sind grosse Beleuchtungsstärken und eine intensive Belüftung mit Sauerstoff notwendig. Wird nach der Vermehrung die Sauerstoffzufuhr stark gedrosselt, so gelingt es manchmal die Normalform der Bakterien zur Ausbildung einer Purpurmembran zu veranlassen, die Rhodopsin und β-Carotin enthält, bevor die Population geschädigt wird. In diesem Fall ist der Mangelstress an Sauerstoff auslösender Faktor für die Induktion der Rhodopsinsynthese. Damit ist es im Rahmen dieser Erfindung jetzt erstmals möglich, die normalerweise durch einen Mangelstress ($O_2$-Limitierung) induzierte Produktionsvariante eines zur Produktion von Sekundärmetaboliten fähigen Mikroorganismus zu stabilisieren, indem man besagten Mikroorganismus in ein statisches Elektrofeld einbringt und dort in einem geeigneten Kultivierungsmedium kultiviert.

Im einzelnen ist das erfindungsgemässe Verfahren dadurch charakterisiert, dass man

a) besagten Mikroorganismus in ein geeignetes Kulturmedium einbringt und
b) diesen im Einflussbereich eines statischen Elektrofeldes kultiviert,
c) die Kultivierung solange fortsetzt, bis sich die Produktionsform ausgebildet und stabilisiert hat und
d) den produzierten Sekundärmetaboliten aus dem Medium oder dem Zellmaterial isoliert.

Die Wahl der für die gewünschte Stabilisierung geeigneten Feldparameter hängt zu einem gewissen Grade von dem verwendeten Mikroorganismus ab. Die bevorzugten Feldstärkewerte liegen zwischen 500 V/cm und 1'500 V/cm, insbesondere zwischen 500 V/cm und 1'000 V/cm.

Besonders bevorzugt im Rahmen dieser Erfindung ist ein Verfahren zur Stabilisierung der Rhodopsin- und/oder β-Carotin-bildenden Variante von Halobacterium halobium. Dies kann erfindungsgemäss dadurch erreicht werden, dass man Bakterien der Normalform von Halobacterium halobium in einen für die Kultivierung dieses Bakteriums geeigneten Mediums in ein statisches Elektrofeld einbringt und dort bei schwachem Licht für einen Zeitraum kultiviert, der für die Induktion und Stabilisierung der Rhodopsin- und/oder β-Carotin-bildenden Variante notwendig ist. Die für die Stabilisierung der Rhodopsin- und/oder β-Carotin-bildenden Variante gewählte Feldstärke des statischen Elektrofeldes beträgt zwischen 500 V/cm und 1'500 V/cm, vorzugsweise aber zwischen 500 V/cm und 1'000 V/cm.

Besondere Massnahmen zur Sicherstellung einer ausreichenden Belüftung sind in diesem Fall nicht notwendig. Die Anwendung des erfindungsgemässen Verfahrens führt dann zur direkten Ausbildung der Rhodopsinform, die auf die übliche Weise durch Animpfen geeigneter Kulturmedien weitervermehrt werden kann.

Die erfindungsgemässe Züchtungsmethode von Halobakterien, insbesondere von Halobakterium halobium, hat gegenüber herkömmlichen Methoden den Vorteil, dass sich die Bakterien schneller vermehren, dass sie in der Zellmembran mindestens 19 % bis 121 % mehr Bakteriorhodopsin und 10 % bis 30 % mehr Biomasse produzieren sowie über einen Gehalt an Vitamin-A-Säure und Benzaldehyd verfügen. Ausserdem behalten Halobakterien, die ihre Genexpression vorausgehend im statischen Elektrofeld stabilisiert haben, ihren Gehalt an Bakteriorhodopsin, Biomasse und Vitamin-A-Säure ohne elektrostatisches Feld für etwa drei Monate bei. Ferner produzieren Bakterien, die dem Elektrofeld ausgesetzt waren, teilweise unterschiedliche Metaboliten, wie beispielsweise Benzaldehyd.

Gegenstand der Erfindung ist zudem ein Bakterienlysat, das aus den - wie oben beschrieben - im statischen Elektrofeld gezüchteten Halobakterien gewonnen wird. Zur Gewinnung des Lysates werden die Bakterien abzentrifugiert und in destilliertem oder demineralisiertem Wasser osmotisch aufgeschlossen. Die entstandene Lösung enthält unter anderem Bakteriorhodopsin, Biomasse, Vitamin-A-Säure und Benzaldehyd. Bakteriorhodopsin und Benzaldehyd werden qualitativ und quantitativ spektroskopisch identifiziert. Die Vitamin-A-Säure wird chromatographisch mittels HPLC nachgewiesen. Die Biomasse wird nach der Menge an Zellproteinen mittels Biuret-Farbreaktion bestimmt. Das erfindungsgemässe Bakterienlysat enthält im Vergleich zum Lysat aus dem Bakterienwildtyp 19 % bis 121 %, insbesondere 50 % bis 100 %, jedoch mindestens 19 % mehr Bakteriorhodopsin und 10 % bis 30 %, insbesondere 15 % bis 25 %, jedoch mindestens 10 % mehr Biomasse sowie einen Gehalt an Vitamin-A-Säure und Benzaldehyd.

Die vorliegende Erfindung betrifft ausserdem Arzneimittelzubereitungen, die als Wirksubstanz

a) Halobakterien, insbesondere Halobakterium halobium, oder
b) standardisiertes Lysat aus Halobakterien, insbesondere Halobakterium halobium, oder
c) Bakteriorhodopsin, Vitamin-A-Säure und Benzaldehyd, oder
d) Bakteriorhodopsin und ein Retinoid, oder
e) Bakteriorhodopsin allein

enthalten.

Es ist vorteilhaft, wenn man diese Arzneimittelzubereitungen mit 13-cis-Vitamin-A-Säure-Derivaten und/oder 13-cis-Vitamin-A-Aldehyden und/oder 13-cis-Vitamin-A-Aldehyd-Derivaten und/oder 13-cis-Vitamin-A-Aldehyd-Schiff'schen Basen und/oder 13-cis-Vitamin-A-Aldehyd-Acetalen und/oder Analogen und/oder cis-/trans-Isomeren der vorstehend genannten Verbindungen anreichert.

Erfindungsgemässe Arzneimittelzubereitungen sind alle geeigneten galenischen Formen, bevorzugt topische Formen, wie zum Beispiel Gele, Lösungen, Emulsionen, Salben, Pasten und Badezusätze, besonders bevorzugt Gele.

Die erfindungsgemässen Arzneimittelzubereitungen enthalten einen oder mehrere der genannten Wirksubstanzen in einer Konzentration von 0,1 % bis 70 %, bevorzugt von 0,5 % bis 50 % und besonders bevorzugt von 1 % bis 10 %.

Erfindungsgemässe Arzneimittelzubereitungen mit einem standardisierten Bakterienlysat von 2 % (siehe Beispiel 6.5.) können weiter verarbeitet werden und daraus - bezogen auf die Ausgangskonzentration von 2 % - Arzneimittelzubereitungen mit einer Konzentration von 0,1 % bis 99 %, bevorzugt von 25 % bis 75 %, besonders bevorzugt von 50 % hergestellt werden.

Zur Herstellung der topischen Arzneimittelzubereitungen eignet sich eine wässrige Lösung des Bakterienlysates, die entweder auf die Haut aufgetragen oder als Badezusatz verwendet wird. Das Lysat kann ebenfalls zu Gelen mit hydrophilen makromolekularen Verbindungen, wie z.B. Gelatine und Celluloseether, zu Emulsionen, unter Verwendung von Öl, arabischem Gummi und Wasser, zu einer hydrophilen Salbengrundlage, z.B. Macrogol- bzw. Polyethylenglykolsalbe, zu Crèmes, die hydrophobe Lipidbestandteile, Wasser und Tenside enthalten, zu Pasten, aus einer hochkonzentrierten Suspensionssalbe, verarbeitet werden. Die Herstellung dieser topischen Arzneimittel erfolgt nach konventionellen Methoden.

Die vorliegende Erfindung umfasst ausserdem die Verwendung der erfindungsgemässen Arzneimittelzubereitungen zur Bekämpfung von Hauterkrankungen, besonders von hyperkeratotischen Erkrankungen, insbesondere von Psoriasis.

Im Arzneimittelschatz sind zahlreiche topische und auch orale Arzneimittel zur Behandlung der Psoriasis verfügbar. Diese Medikamente bringen allerdings nur als Langzeittherapeutika eine symptomatische Linderung der Beschwerden, sind meist mit Nebenwirkungen verbunden und bei Abbruch der Behandlung verschlimmert sich der Zustand des Patienten innert kurzer Zeit wieder.

Der vorliegenden Erfindung liegt die Aufgabe zugrunde, Arzneimittel zu entwickeln, die eine Psoriasis vollständig heilen oder zumindest nach Abbruch der Medikation eine lange beschwerdefreie Zeit garantieren sowie frei von Nebenwirkungen oder nebenwirkungsarm sind.

In Vorversuchen wurden Psoriatiker erfindungsgemäss mit einem 2 %igen Lysat des Halobakterium halobium behandelt. Hierbei wurden mehrere Schuppenbereiche mit dem Bakterienlysat bepinselt und anschliessend einer 40-minütigen Bestrahlung mit einer 300 Watt-Lampe ausgesetzt. Während dieser Zeit wurden die Hautbezirke weitere fünf- bis sechsmal mit dem Bakterienlysat bepinselt. Die Behandlung wurde täglich über einen Zeitraum von 200 Tagen durchgeführt. Im Vergleich zu den unbehandelten Kontrollen heilten die behandelten Hautbezirke vollständige ab und bildeten auch nach 6 Jahren keine Rezidiven.

Die erfindungsgemässen Arzneimittelzubereitungen werden allgemein zur Bekämpfung von Hauterkrankungen, besonders von hyperkeratotischen Prozessen, insbesondere der Psoriasis eingesetzt. Zur Behandlung der Psoriasis wird die Arzneimittelzubereitung zwei- bis dreimal täglich auf die betroffenen Hautstellen aufgetragen, bei Bedarf kann zusätzlich nach Auftragen des Arzneimittels mit einer 300 Watt Glühbirne während 20 bis 60 Minuten, vorzugsweise während 40 Minuten, bestrahlt werden. Die Anwendung von hautschädigenden UV-Strahlen, wie sie üblicherweise als Begleittherapie von Psoriasis eingesetzt werden, erübrigt sich.

Die Anwendung des erfindungsgemässen Verfahrens führt noch zu einem weiteren Phänomen, das für die praktische Anwendung von Bedeutung ist. Durch die Verdunstung des Wassers in den Kulturschalen entstehen Kochsalzkristalle, die Rhodopsin-bildende Halobakterien eingeschlossen enthalten. Überraschderweise überleben und vermehren sich diese im Innern der Kristalle, wenn der Kristall in einem E-Feld gehalten wird. Auch eine wochenlange Kälteperiode im Tiefkühlschrank wird unbeschadet überstanden. Eine Nachkultur ist durchaus möglich, wenn der Kristall in einem Nährmedium aufgelöst wird.

Die gleichen Beobachtungen lassen sich auch bei mittels des erfindungsgemässen Verfahrens an hohe Salzkonzentrationen adaptieren ubiquitären Bakterien machen, die somit ebenfalls eingeschlossen in Salzkristalle aufbewahrt werden können. Das erfindungsgemässe Verfahren ist somit im einzelnen dadurch charakterisiert, dass man

a) besagte Bakterien in einer hochkonzentrierten Salzlösung inkubiert,
b) diese anschliessend in ein statisches Elektrofeld einbringt,
c) das Lösungsmittel allmählich verdunstet und
d) die Bakterien in den auskristallisierten Salzkristallen einschliesst.

Damit liegt nunmehr ein sehr einfaches und - aus praktischen Erwägungen heraus betrachtet - effizientes Aufbewahrungssystem für Halobakterien sowie für an hohe Salzkonzentrationen adaptierte ubiquitäre Bakterien vor. Für die Kultivierung und Vermehrung dieser Bakterien genügt es dann, Salzkristalle, die diese Bakterien in lebensfähiger Form enthalten, in einem geeigneten Kultivierungsmedium zu lösen und anschliessend unter an sich bekannten Kultivierungsbedingungen zu kultivieren.

Eine weitere Form von Stress ergibt sich beim Zusammenleben verschiedener Organismen in einer gemischten Population.

Auch auf dieses System kann das erfindungsgemässe Verfahren modifizierend einwirken, indem unter dem Einfluss eines statischen Elektrofeldes bestimmte Mitglieder einer Mischpopulation in ihrem Wachstum und in ihrer Entwicklung gefördert werden und somit dem Konkurrenzdruck besser widerstehen können. Dies geschieht im allgemeinen auf Kosten eines oder mehrerer anderer Mitglieder der Mischpopulation.

Beispielhaft kann dies an einer Bakterien-Mischkultur demonstriert werden, bestehend auch E. coli 205, K. pneumoniae 327, P. aeruginosa, S. aureus 10B, S pyogenes L-15 sowie Bacillus subtilis MX-1. Wird diese Mischkultur im Einflussbereich eines statischen Elektrofeldes kultiviert, so kommt es zu signifikanten Verschiebungen in der Populationsstruktur, wie z.B. einer starken Zunahme von E. coli auf Kosten von K. pneumoniae.

Es ist somit möglich, mit Hilfe des erfindungsgemässen Verfahrens die Zusammensetzung einer Mischpopulation signifikant zu verändern, was in der Praxis mannigfache Anwendungsmöglichkeiten eröffnet.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.

**Nichtlimitierte Ausführungsbeispiele**

Aufbau des statischen Elektrofeldes (nachfolgend zitiert als „Versuchsaufbau")

Alle im folgenden beschriebenen Versuche werden in statischen Elektrofeldern durchgeführt, die zwischen den Platten eines Kondensators aufgebaut werden.

Die elektrische Feldstärke ist durch die Beziehung

$$E = \frac{U}{d'}$$

gegeben, worin U die Spannungsdifferenz zwischen den Kondensatorplatten und d' den Plattenabstand des Kondensators bedeuten. Die Spannung wird mit Hilfe eines Hochspannungsgenerators erzeugt, der auf dem Transformatorprinzip mit Gleichrichter basiert, wobei in der Regel Spannungsdifferenzen zwischen 500 Volt und 12'000 Volt verwendet werden. Der Plattenabstand des Kondensators richtet sich nach den Dimensionen der in den einzelnen Versuchen verwendeten Probengefässe. In der Regel werden die variablen Parameter U und d' so gewählt, dass das statische Elektrofeld Feldstärkewerte zwischen 250 V/cm und 1500 V/cm aufweist.

1. Beispiel: Keimung im statischen Elektrofeld

1.1. Gartenkresse

Eine abgezählte Anzahl von Kressesamen (140 Stück pro Versuchsansatz) wird in Petrischalen auf Filterpapier verteilt, mit 5 ml Wasser versetzt und mit Parafilm verschlossen. Eine der Petrischalen wird während der Keimungsphase (ca. 5 Tage) in die zuvor beschriebene Versuchsanordnung (siehe „Versuchsaufbau") eingebracht und dort einem starken statischen Elektrofeld mit Feldstärkewerten von 750 V/cm ausgesetzt. Die negativ geladene Platte des Kondensators bildet dabei den Deckel der Versuchszelle. Die zweite Petrischale bleibt ausserhalb des Einflussbereiches des statischen Elektrofeldes und dient als Kontrolle.

Um eine unkontrollierte Einflussnahme des Lichtes auf die Keimung auszuschliessen, wird der Versuch unter standardisierten Licht- und Temperaturverhältnissen in einer Dunkelkammer durchgeführt, deren Innenraum von einer 100 Watt Pflanzenlampe in einem Abstand von 28 cm zu den Oberflächen der Versuchszellen beleuchtet wird. Die Temperatur in der Dunkelkammer beträgt zwischen 23° C und 24° C.

Die Keimungsrate der eingesetzten Kressesamen wird nach der Entwicklung des Hypokotyls und der Keimblätter bestimmt. Sie beträgt für die im statischen Elektrofeld gekeimten Samen im Mittel 83 %. Im Blindversuch ohne statisches Elektrofeld keimen von der gleichen Anzahl Samen dagegen nur durchschnittlich 21 %. Die anschliessende Nachaussaat in Erde am Tageslicht zeigt, dass alle Samen in gleicher Weise keimfähig sind.

## 1.2. Weizen

Die Keimungsversuche mit Weizen werden in genau gleicher Weise wie zuvor für Kresse beschrieben (vgl. Beispiel 1.1.) durchgeführt. Bei den verwendeten Weizensorten handelt es sich um einen Weichweizen (Anza-Weizen, Kanada) und einen Hartweizen (Raineri-Weizen, Italien). Bei den mit Weizen durchgeführten Versuchen beträgt die Anzahl der getesteten Samen 30 pro Petrischale. Diese werden in Petrischalen mit je 15 ml Wasser versetzt, mit Parafilm verschlossen und für einen Zeitraum von 1 bis 7 Tagen in Wasser angekeimt, wobei die Samen der Versuchspflanzen wiederum einem starken statischen Elektrofeld ausgesetzt werden. Die Feldstärkewerte liegen auch in diesem Fall bei 750 V/cm.

Die übrigen Bedingungen sind wie folgt standardisiert:

Beleuchtungsstärke:  100 Watt Pflanzenlampe (Osram Concentra R 95 Natura) in 80 cm Distanz entsprechend 133 $\mu$Watt/cm$^2$.

Temperatur:  23 bis 24° C

## Hartweizen (Raineri)

Die Keimungsversuche mit Winterweizen (Raineri) machen deutlich, dass sowohl die Keimungsrate als auch die Anzahl und Länge der Wurzeln sowie die Länge des Epikotyls unter dem Einfluss eines statischen Elektrofeldes stark zunehmen im Vergleich zur Kontrolle (siehe Tabelle 1). Bei Fortführung des Versuches kann man zeigen, dass dieser Vorsprung auch nach der Aussaat bis zur Reife der Pflanzen erhalten bleibt.

Tabelle 1

| Vergleich der Keimungs- und Wachstumseffizienz von im statischen Elektrofeld behandelten sowie unbehandelten Weizensamen (Raineri): | | | |
|---|---|---|---|
| | ohne E-Feld | mit E-Feld | Δ % |
| Keimung | 19 | 26 | 137 % |
| Anzahl Wurzeln | 106 | 137 | 129 % |
| Wurzel-Länge (mm) | 112 | 188 | 168 % |
| Epikotyl-Länge (mm) | 222 | 296 | 133 % |

## Vergleich Anza Weizen und Raineri-Weizen

Anza-Weizen (Weichweizen Kanada) und Raineri-Weizen (Hartweizen Italien) verhalten sich differenziert.

Tabelle 2

| Vergleich des Einflusses eines statischen Elektrofeldes auf Samen von Anza-Weizen und Raineri-Weizen: | | | | | | |
|---|---|---|---|---|---|---|
| | Anza-Weizen | | | Raineri-Weizen | | |
| | o/Feld | m/Feld | Δ % | o/Feld | m/Feld | Δ % |
| Keimung | 2 | 2 | 100 % | 3 | 4 | 133 % |
| Anzahl Wurzeln | 7 | 8 | 114 % | 11 | 15 | 136 % |
| Wurzel-Länge (mm) | 140 | 160 | 114 % | 140 | 380 | 270 % |
| Epikotyl-Länge (mm) | 220 | 170 | 80 % | 110 | 490 | 460 % |

Anza-Weizen wird weit weniger begünstigt: Die Keimung und das Wurzelwachstum bleiben etwa gleich; das Epikotyl wird eher gehemmt (siehe Tabelle 2). In einem Feldversuch zeigt sich, dass Anza-Weizen, der in einem Elektrofeld angekeimt wurde, in der Anfangsphase schneller wächst als der ohne Elektrofeld angekeimte, dass aber bis zur Reife die Unterschiede verschwinden. Dennoch kann aus diesen Untersuchungen gesamthaft der Schluss gezogen werden, dass ein Elektrofeld sowohl die Keimungsrate wie auch das Wachstumsverhalten beeinflusst.

1.3. Mais

Analog zu der in Beispiel 1.2. für Weizen beschriebenen Verfahrensweise werden je 10 Samen der Mais-Sorte FMI A 188 bei einer Feldstärke von 1'500 V/cm in einem statischen Elektrofeld zur Keimung gebracht. Nach einer Behandlungsdauer von 6 Tagen lässt sich bei den im statischen Elektrofeld behandelten Proben ebenfalls eine erhöhte Keimungsrate gegenüber den Kontrollen nachweisen.

Tabelle 3 a

| Vergleich der Keimungs- und Wachstumseffizienz von im statischen Elektrofeld behandelten sowie von unbehandelten Mais-Samen: | | |
|---|---|---|
| | ohne E-Feld | mit E-Feld |
| Keimung | 16 | 20 |
| Epikotyl-Länge (mm) | 0 | 30 |

Die so auf die beschriebene Weise vorgekeimten Samen werden anschliessend in 5 l Töpfen in standardisierte Erde [40 % Landerde, 50 % Torf (TSK-1; Floratorf), 10 % Sand] überführt und wie normale Keimlinge weitergezogen. Bereits nach 4 Tagen lassen sich deutliche Unterschiede im Auflaufverhalten sowie der Wachstumsgeschwindigkeit zwischen Kontroll- und Versuchspflanzen erkennen.

Tabelle 3 b

| Unterschiede im Auflaufverhalten und der Wachstumsgeschwindigkeit zwischen behandelten und unbehandelten Maiskeimlingen: | | |
|---|---|---|
| | ohne E-Feld | mit E-Feld |
| Auflauf | 9 | 20 |
| Blattausbildung | 1-2 | 3-4 |

## 2. Beispiel: Morphologische Veränderung

Adulte Pflanzen, die aus Samen hervorgegangen sind, welche unter den zuvor beschriebenen Bedingungen im statischen Elektrofeld angekeimt wurden, weisen zum Teil beträchtliche morphologische Veränderungen gegenüber den Kontrollpflanzen auf. Diese Veränderungen betreffen:

a) mehrere Stengel mit mehreren Ähren (2 bis 7)
b) veränderte Blattstellung
c) Ausbildung von Rispen mit einer Vielzahl von Ährchen
d) Buschformen, Kriechformen

### 2.1. Weizen

5 Weizenkörner (Raineri) werden in eine Petrischale mit 10 ml Wasser eingetragen. Die Schale wird mit Parafilmstreifen verschlossen und in der oben beschriebenen Versuchsanordnung (vgl. „Versuchsaufbau") 8 Tage bei einer Feldstärke von 1'500 V/cm gekeimt. Die Keimlinge werden anschliessend mit ½ l Töpfe mit steriler Ackererde (vgl. Beispiel 1.3.) eingepflanzt und dort normal weiterkultiviert. Bereits in der Keimungsphase löst das E-Feld ein verstärktes Wurzelwachstum aus, das sich in Anzahl und Grösse der gebildeten Wurzeln widerspiegelt sowie ein verstärktes Wachstum des Epikotyls zur Folge hat. Nach einer Kultivierungsdauer von 8 bis 10 Wochen weisen die eingepflanzten Keimlinge eine im Vergleich zu den Kontrollpflanzen deutlich abweichende Morphologie auf:

1. eine grössere Anzahl Stengel (6,8 im Vergleich zu 4,8)
2. eine um den gleichen Betrag vermehrte Anzahl Ähren
3. grössere Ähren (Körnerzahl 38 im Vergleich zu 32)
4. mitunter tritt ein Habitus auf, der genetischen Vorfahren des Weizens gleicht: beispielsweise rispengrasartige Anordnung kleiner Ährchen und kleine schmale Blätter.

### 2.2. Mais

20 Maiskörner (FMI A 188) werden in einer Petrischale mit 15 ml Wasser überschichtet. Die Schale wird mit Parafilm verschlossen und in der oben beschriebenen Versuchsanordnung (vgl. „Versuchsaufbau") 8 Tage bei einer Feldstärke von 1500 V/cm gekeimt. Die Keimlinge, die ebenfalls ein verstärktes Wurzel- und Epikotylwachstum aufweisen, werden anschliessend in 5 l Töpfen in sterile Ackererde (vgl. Beispiel 1.3.) eingepflanzt und im Gewächshaus normal weiterkultiviert. Wöchentlich wird die Erde mit Standarddünger (Wuxal, Fa. Maag, CH) begossen.

Nach einer Kultivierungsdauer von 14 Wochen zeigen die Keimlinge eine im Vergleich zu den Kontrollen deutlich abweichende Morphologie:

1. eine grössere Anzahl Kolben/Pflanze (3-6 gegenüber 1-2)
2. gedrungener Habitus im Vergleich zur Norm (breite Blätter, dicker Stengel)
3. eine von der Norm abweichende Positionierung der Kolben (Kolben am oberen Stengelende anstatt in der Blattachse)
4. Dreifachkolben in ährenartiger Anordnung, wobei die beiden Nebenkolben in nur 5 Tagen voll entwickelt sind und diese Kolben noch einmal weitere Kolben ausbilden
5. Ausbildung mehrerer Stengel.

### 2.3. Farn

In einer Petrischale wird ein Cellulosegewebe ['Lens clean' Papier (Spectra Physics)] eingelegt und mit 2 ml Evianwasser [Mineralwasser, S.A. Des eaux minérales d'Evian, (Evian, France)] übergossen. Auf dieses Papier werden Sporen von Wurmfarn, die durch Abklopfen von Farnblättern gewonnen werden, in grösserer Zahl aufgetragen. Die Petrischale wird mit Parafilm verschlossen und in der oben (vgl. „Versuchsaufbau") beschriebenen Apparatur einem statischen E-Feld von 1'500 V/cm ausgesetzt. Nach 6-7 Wochen beginnen die Sporen zu keimen und bilden im Verlauf von etwa 7 Monaten ein Protallium aus, das sich bis zum 12. Monat voll entwickelt. In diesem Stadium werden die Pflanzen in sterile, standardisierte Ackererde (vgl. Beispiel 1.3.) übertragen. Dazu wird der mit Protallien belegte Teil des Papiers ausgeschnitten und auf die Oberfläche der mit viel Wasser benetzten Erdschicht aufgelegt. Im Verlaufe von 6 Wochen findet auf den Protallien die Befruchtung statt und die ersten Farnpflänzchen beginnen sich zu entwickeln. Die so entstandenen Pflanzen unterscheiden sich morphologisch ganz bedeutend von den Kontrollpflanzen. Die Blätter zeigen einen ganzrandigen wenn auch stark gefalteten Habitus; eine Blatteilung findet nicht statt. Die Anordnung der Blätter erscheint im Vergleich zum normalen Wachstum zentriert anstatt ausladend.

2.4. Hyoscyamus muticus L

Je 5 Antheren von Hyoscyamus muticus L. werden in 6 cm Petrischalen auf sterilem Agar (Nitch, Science, 1969, 163, 85-87) angezogen. Die Schalen werden mit Parafilm steril verschlossen und in der oben beschriebenen Versuchsanordnung (vgl. „Versuchsaufbau") bei Feldstärkewerten von 2'000 V/cm mehrere Wochen kultiviert. Nach einer Kultivierungsdauer von ca. 6 Wochen entwickeln sich die ersten haploiden Pflänzchen.

Auch in diesem Fall besitzen die im Elektrofeld kultivierten Pflänzchen einen Entwicklungsvorsprung gegenüber den Kontrollen. Von je 45 Antheren entwickeln sich im Elektrofeld bis zu diesem Zeitpunkt 15 zu Pflänzchen, während dies bei den Kontrollen nur für 10 zutrifft.

Die Pflänzchen werden nunmehr in grössere geschlossene Kunststoffgefässe (Durchmesser 9 cm, Höhe 6 cm) in frischen sterilen Agar überpflanzt. Die im Elektrofeld angezogenen werden weiterhin einem E-Feld von einer Stärke von 1'500 V/cm ausgesetzt. Schon jetzt zeigen die Pflänzchen deutliche morphologische Unterschiede, indem diejenigen ohne E-Feld runde, diejenigen mit E-Feld lanzettförmige Blätter ausbilden.

Nach weiteren 7 Wochen werden die Pflanzen in Töpfe mit Vermikulit (exfolierter Glimmer, Fa. Vermica AG, Bözen, CH) überführt und bei einer Temperatur von 26° C bis 27° C und einer relativen Luftfeuchtigkeit von 60 % in einer Klimakammer weiterkultiviert. Die Beleuchtungsstärke beträgt 10'000 Lux, wobei ein Tag/Nachtrhythmus von 12 h Tag-/12 h Nacht eingehalten wird.

Die weitere Kultivierung der Versuchspflanzen erfolgt jetzt ohne den Einfluss eines statischen Elektrofeldes. Nach 10 Wochen hat sich der Habitus der im E-Feld angezogenen Pflanzen gegenüber den Kontrollen noch einmal deutlich verändert. Während die Blätter der Kontrollpflanzen buschartig angeordnet sind, lässt sich bei den im Feld kultivierten Pflanzen die Ausbildung eines Blattstieles mit quirlständigen Blättern beobachten sowie die beginnende Ausbildung von Blütenknospen. In diesem Stand werden die Pflanzen in normale Pflanzenerde übertragen ins Gewächshaus überführt und dort weiterkultiviert.

Nach weiteren 3 Monaten zeigen die Kontrollpflanzen einen buschartigen Habitus mit niedrigem Stiel der noch keine Blüten trägt. Die im Feld behandelten Pflanzen dagegen sind dreistenglig. Mehrere sind bereits verblüht und tragen Fruchtansätze. Die daraus entstehenden Fruchtkapseln werden gewonnen und die Samen gesammelt. Die Samen der unbehandelten Pflanzen sind normalfarbig, die im Elektrofeld gezogenen weiss. Letzteres ist ein bekanntes Merkmal für Haploidie. Ein weiteres Merkmal für Haploidie ist nach Wernike et al. (Plant Scienc. Lett., 1979, 15, 239-249) die Anzahl Chloroplasten pro Blattzelle. Die Auszählung ergibt für die unbehandelten Pflanzen ein Verhältnis von durchschnittlich 13, für die Versuchspflanzen dagegen durchschnittlich 7,8. Die im Elektrofeld gezogenen Pflanzen weisen damit vorwiegend typisch haploide Merkmale auf im Gegensatz zu den Kontrollen.

3. Beispiel: Veränderungen des Wachstums

Neben morphologischen Veränderungen findet man bei adulten Pflanzen, die aus Samen hervorgegangen sind, die unter dem Einfluss eines statischen Elektrofeldes angekeimt wurden, auch eine grosse Variabilität im Wachstumsverhalten:

a) erhöhte Biomasse (10 bis 100 %)
b) Ertragssteigerung, verteilt auf mehrere Ähren (30 bis 120 %)
c) stark beschleunigtes Wachstum bei Ausbildung eines zweiten Stengels (innerhalb von 4 Wochen)
d) perennierende Lebensweise der aus behandelten Samen hervorgegangenen Pflanzen

4. Beispiel: Nachkommen

Die Samen der zuvor beschriebenen Versuchspflanzen bleiben voll keimfähig, die Keimungsrate liegt bei 100 %. Die zuvor beschriebenen morphologischen Veränderungen sowie die Veränderungen im Wachstumsverhalten bleiben auch in der Folgegeneration zum Teil erhalten. Diese Pflanzen können daher zur Züchtung neuer und verbesserter Pflanzensorten herangezogen werden (Saatgutverbesserung).

5. Beispiel: Veränderung der Genexpression

Zur Überprüfung eventueller Unterschiede in der Genexpression behandelter Pflanzen werden die Proteinmuster von Weizenkörnern aus Kontroll- und Versuchspflanzen mit Hilfe Gel-elektrophoretischer (PAGE) Verfahren untersucht (Stegemann H. et al., AGE Manual, 1986; Stegmann H., Z. Anal. Chem., 1970, 252; 165-169). Bei der Versuchspflanze handelt es sich um einen Raineri-Weizen, der aufgrund der Keimung im statischen Elektrofeld neben dem normalen Haupttrieb zusätzlich noch einen Seitentrieb entwickelt hat. Es werden sowohl die Proteinmuster von Körnern aus dem Haupttrieb als auch aus dem Seitentrieb untersucht sowie als Kontrolle Körner nicht behandelter Raineri-Pflanzen.

5.1. SDS PAGE der wasserlöslichen Proteine (Albumine)

Weizenkörner werden zu Mehl vermahlen und mit Wasser extrahiert. Die Untersuchung der Proteinextrakte erfolgt mittels SDS PAGE.

a) Prüflösungen, Inkubation

Die für die elektrophoretische Untersuchung vorgesehenen Proteinextrakte werden wie folgt mit SDS inkubiert und für die Applikation vorbereitet.

Puffer pH 7,1          1,226 g TRIS-Puffer
                         12,366 g Borsäure ($H_3BO_3$)
                         zusammen in Wasser lösen, auf 200 ml einstellen
Inkubationslösung:    5 % SDS und 5 % 2-Mercaptoethanol in Puffer pH 7,1
Ansatz:              400 µl Proteinextrakt + 100 µl Inkubationslösung mischen
Inkubationszeit:       3 Minuten in siedendem Wasserbad
Applikationslösung:   Zu 500 µl inkubierte Proteinlösung ca. 100 mg Zucker (Saccharose puriss; Fluka) und 1 Tropfen
                         0,1 %ige wässrige Amidoschwarzlösung zusetzen und mischen.
Applikation:           je 40 µl.

b) Elektrophoresepuffer

TRIS/Borsäure pH 7,1:  6,13 g TRIS-Puffer
                         61,83 g Borsäure ($H_3BO_3$)
                         5,00 g SDS (Dodecylsulfat · Na)
                         zusammen in Wasser lösen, auf 5000 ml auffüllen

c) Elektrophorese

Temperatur:             0-2° C
Pufferumwälzung:      wird durchgeführt
Spannung und Laufzeit:  400 V, 105-120 Minuten

d) Nachweis

Proteinfärbung mit Coomassie-Brillant Blue R-250

5.2. SDS-PAGE der in Tris/Borat-Puffer löslichen Proteine (Globuline)

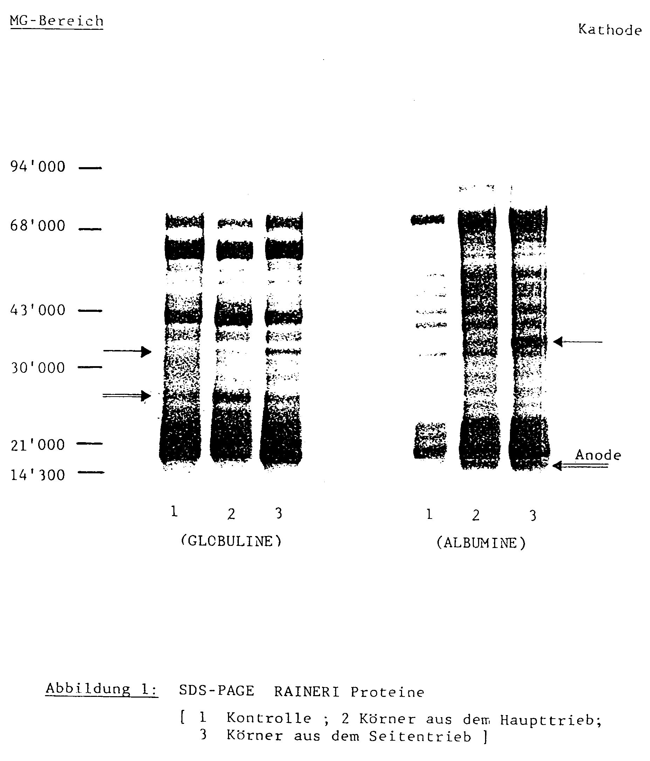

Aus dem in Arbeitsschritt 5.1. erhaltenen Sediment werden weitere Proteine (Globuline) mit TRIS/Borat-Puffer extrahiert und ebenfalls mittels SDS-PAGE (vgl. Abschnitt 5.1.) analysiert.
Eindeutige Unterschiede, wie z.B. Feststellung von fehlenden resp. Auftreten von zusätzlichen Zonen oder besonders starke Intensitätsunterschiede bei Zonen, zeigt die SDS-PAGE der wasserlöslichen und der salzlöslichen Proteine. Diese Unterschiede werden durch Wiederholung der Extraktionen und nochmalige Prüfung aller Extrakte nebeneinander bestätigt und sind in Abbildung 1 wiedergegeben. Auch bei den anderen getesteten Methoden (Normal-PAGE, Esterasen, IEF, Gliadine) lassen sich geringe Intensitätsunterschiede einzelner Zonen feststellen.

5.3. Ergebnisse

SDS-PAGE der wasserlöslichen Proteine (Albumine)

Die Probe aus Körnern des Haupttriebes (2) unterscheidet sich von der unbehandelten Kontrolle durch das Auftreten seiner schwachen zusätzlichen Proteinzone im MG-Bereich bei 29'000 (←). In der Probe des Seitentriebes (3) tritt diese zusätzliche Zone sogar relativ stark auf. Im MG-Bereich 13'000 ist in beiden Proben eine Zone zu beobachten, die in der Probe der unbehandelten Kontrolle fehlt (⇐).

SDS-PAGE der in TRIS/Borat-Puffer löslichen Proteine (Globuline)

Auch in diesem Pherogramm finden sich die deutlichsten Unterschiede hauptsächlich im MG-Bereich zwischen 14'000 und 30'000. Im MG-Bereich um 28'000 tritt bei Proben aus den Versuchspflanzen (2, 3) wie bei den Albuminen eine zusätzliche schwache Zone auf ($\rightarrow$), und bei MG ca. 24'000 ist in der Probe aus dem Haupttrieb (2) eine starke Zone zu beobachten, die in den beiden anderen Proben nur sehr schwach zu erkennen ist ($\Rightarrow$).

6. Beispiel: Einfluss des statischen Elektrofeldes auf das Stressverhalten

6.1. Verzögertes Einsetzen der Seneszens bei Grünalgen

Durch Ausstrich auf Agarnährböden in Petrischalen erhaltene Grünalgenkulturen werden bei ungenügender Beleuchtung (133 $\mu$Watt/cm$^2$) in der oben beschriebenen Versuchsanordnung (vgl. „Versuchsaufbau") entwickelt. Sowohl bei einer Feldstärke von 1'500 V/cm wie bei einer solchen von 750 V/cm überdauern die Kulturen wesentlich länger als Vergleichsmaterial ohne Elektrofeld.
Letzteres zeigt bereits nach 1 Monat deutliche Mangelerscheinungen und ist nach 2 Monaten braun. Im gleichen Zeitraum sind demgegenüber im E-Feld gezogene Kulturen normal entwickelt.

6.2. Erhöhte Salzresistenz ubiquitärer Bakterien

Ein zufälliges Gemisch ubiquitärer Bakterien aus einer Bodenprobe wird in Petrischalen 5 Tage in Wasser bei Zimmertemperatur angezogen. Aus dieser Suspension werden 6 Petrischalen, die je 15 ml eines verdünnten Meerwassermediums (vgl. Tabelle 4; Verdünnung 1:2, 1:4, 1:8) enthalten, zu je 1 ml angeimpft. 3 Schalen werden in der oben beschriebenen Versuchsanordnung (vgl. „Versuchsaufbau") einem statischen Elektrofeld von 1'500 V/cm ausgesetzt, die übrigen dienen als Kontrollen. Nach 7 Tagen zeigen die Schalen im E-Feld eine deutliche Zunahme der Bakterienpopulation, wohingegen die Kontrollschalen keine Entwicklung erkennen lassen. Das E-Feld hilft den Salzstress überwinden.
In einer zweiten Adaptationsstufe werden Bakterien aus der bei einer Verdünnung von 1:2 im E-Feld gewachsenen Kultur entnommen. Diese dienen als Ausgangsmaterial um eine weitere Petrischale anzuimpfen, die nunmehr das Kulturmedium gemäss Tabelle 4 enthält. 1 ml dieser Suspension wird in 15 ml Meerwasser-Medium übertragen und die Petrischale wiederum in der oben beschriebenen Versuchsanordnung (vgl. „Versuchsaufbau") einem statischen Elektrofeld von 1'500 V/cm ausgesetzt. Innerhalb von 3 Wochen entwickelt sich die Kultur vollständig, d.h. bis zur maximalen Gleichgewichtspopulation. Damit ist ein ubiquitäres Bakteriengemisch in 2 Schritten vom Süsswasser an eine gesättigte Salzlösung angepasst.

Tabelle 4

| Meerwasser-Kulturmedium | |
|---|---|
| Salze (Menge pro 1 Liter Lösung) | |
| NaOH | 7-8 g |
| NaCl | 250 g |
| KCl | 2 g |
| $CaCl_2$ | 7 mg |
| DL-Malonsäure (Apfelsäure) | 15 g |
| $Na_3$-citrat | 0,5 g |
| Glycerin | 1 ml |
| Casaminosäuren | 7,5 g |
| Phosphatpufferlösung | 1 ml |
| Vitamine | 1 ml |
| Spurenelemente | 1 ml |
| in $H_2O$ lösen und anschliessend 9 g $MgSO_4$ Anhydrid zugeben und mit NaOH titrieren auf pH 6,8 - 7,0. | |
| Phosphatpufferlösung | |
| $K_2HPO_4$ | 50 g/l |
| $KH_2PO_4$ | 50 g/l |
| Vitaminlösung | |
| Folsäure | 100 mg/100 ml |
| Thiamin | 100 mg/100 ml |
| Biotin | 100 mg/100 ml |
| Spurenelemente | |
| $ZnSO_4$ | 44 mg/100 ml |
| $MnCl_2$ | 30 mg/100 ml |
| $Fe(II)Cl_2$ | 230 mg/100 ml |

6.3. Einschluss adaptierter Bakterien in Kochsalzkristalle

Lässt man während oder nach der Entwicklung einer Bakterienkultur gemäss Beispiel 6.2. eine geringe Menge Wasser verdunsten, so beginnt die Meerwasserlösung auszukristallisieren. Erfolgt dieser Prozess im statischen Elektrofeld gemäss oben beschriebener Versuchsanordnung (vgl. „Versuchsaufbau"), so kristallisiert vornehmlich Kochsalz aus. Im Gegensatz zur normalen Kristalltracht eines Würfels entstehen jedoch Oktaeder mit einer Körperachse in Richtung des Elektrofeldes. Im Innern eines solchen Kristalles finden sich Bakterien eingeschlossen. Teilweise sind diese vom Salz voll umschlossen, teils halten sie sich in Kammern auf, in denen sie ihre Beweglichkeit und Lebensaktivität über mehrere Jahre aufrecht erhalten können. Sie sind darüber hinaus fähig, die Kammern zu erweitern, und sich zu vermehren. In dieser Form können sie auch unbeschadet einen Kälteschock bei -35° C über Wochen überstehen. Löst man die Kristalle anschliessend oder auch nach Jahren in Meerwasser oder in einem anderen geeigneten Medium wieder auf, so erweisen sie sich als voll vital.

6.4. Halobakterien - Ausbildung der Rhodopsinform

Halobakterien existieren in zwei Formen, einer Normalform und einer Rhodopsin-bildenden Form, die als Produzent von Rhodopsin und $\beta$-Carotin gesucht ist.

Ein Stamm normaler Halobakterien (Halobacterium halobium-Wildtyp HHBM, Rechovot, Israel) dient als Ausgangsmaterial zum Animpfen von 20 ml eines Meerwassermediums (vgl. Tabelle 4) das in einer Petrischale mit Parafilm eingeschlossen wird. Als Impfmaterial verwendet man 100 µl einer Stammlösung, die 50 mg Halobakterien pro 10 ml Meerwassermedium enthält. Die Versuchsgefässe werden in ein statisches Elektrofeld eingebracht und dort bei Feldstärkewerten zwischen 750 V/cm und 1500 V/cm bei einer Beleuchtungsstärke von 130 µW cm$^{-2}$ inkubiert. Unter diesen Bedingungen bilden die Halobakterien innerhalb von 8 bis 14 Tagen die Rhodopsinform aus und beginnen mit der Produktion von Rhodopsin und $\beta$-Carotin. Nach 3 bis 4 Wochen entsteht eine gesättigte Population von Halobakterien mit Purpurmembran. Auf dieser Stammlösung können fortlaufend Kulturflaschen angeimpft werden. Man benötigt 100 µl der beschriebenen Stammlösung und 500 ml Meerwasser. Die Flaschen werden mit Parafilm verschlossen - also nicht mit dem Deckel verschraubt - und senkrecht stehend in der oben beschriebenen Versuchsanordnung (vgl. „Versuchsaufbau") bei Zimmertemperatur und 750 V/cm bebrütet. Nach 6 Wochen wird eine maximale Gleichgewichtspopulation erreicht. Rhodopsin kann daraus in an sich bekannter Weise durch Abzentrifugieren der Bakterien und anschliessendem Aufnehmen derselben in dest. Wasser gewonnen werden.

Der Prozess kann auch kontinuierlich gestaltet werden. Man saugt dazu die unten im Gefäss mit Bakterien gesättigte Lösung ab und setzt oben gleichzeitig dieselbe Menge frischen Meerwassers zu. In grösseren Apparaturen empfiehlt sich die Zufuhr geringer Mengen Luft.

Nachweis der Rhodopsinform

Zum Nachweis, dass unter Feldbedingungen tatsächlich die Rhodopsinform auf direktem Wege entstanden ist, werden die Bakterienkulturen abzentrifugiert und die so gewonnenen Bakterienzellen in destilliertem Wasser resuspendiert.

Dies führt zur Lyse der Bakterienzellen und zur Freisetzung von Rhodopsin und $\beta$-Carotin. Das auf diese Weise aus den Zellen freigesetzte Rhodopsin und $\beta$-Carotin kann dann mit Hilfe spektroskopischer Verfahren identifiziert werden.

6.5. Standardisiertes Bakterienlysat 2% zur Behandlung von Hauterkrankungen

Herstellung des standardisierten Bakterienlysates 2%:

0,5 Liter Nährlösung, enthaltend das erfindungsgemässe Halobakterium halobium, werden während 25 Minuten bei 5'500 U/Min. zentrifugiert. Das erhaltene Bakterienpellet wird mit einer entsprechenden Menge an demineralisiertem Wasser versetzt, so dass eine 2 %ige Bakterienlysat-Lösung entsteht. Das Bakterienlysat wird auf dem Magnetrührer bei 700 U/Min. gerührt, bis die Lösung homogen ist (ca. 10 Min.).

6.6. Bakterienlysat-Gel 50 % zur Behandlung von Hauterkrankungen

Zusammensetzung für 100 g Gel:

| | |
|---|---|
| standardisiertes Bakterienlysat 2% | 50,0 g |
| Na-Carboxymethylcellulose (Carbopol 980) | 1,5 g |
| Isopropylmyristat | 5,0 g |
| Polysorbat 80 | 5,0 g |
| Isopropanol | 30,0 g |
| Tromethamin 1 M | 7,5 g |

Ansatz 1 : 50 g standardisiertes Bakterienlysat 2% auf dem Magnetrührer bei 700 U/Min. homogen halten. Unter Rühren nach und nach Na-Carboxymethylcellulose zugeben und gut mischen (ca. 10 Min.). Anschliessend 30 Minuten quellen lassen.

Ansatz 2: Isopropylmyristat, Polysorbat 80 und Isopropanol abwiegen und im Becherglas auf Magnetrührer mit 700 U/Min. mischen.

Fertigstellung des Gels: Ansatz 2 in Ansatz 1 geben und mit Magnetrührer bei 700 U/Min. mischen, bis das Gel homogen ist. Unter Weiterrühren das Gel mit 1 M Tromethamin auf einen pH-Wert von 6-7 titrieren.

6.7. Einschluss der Rhodopsinform von Halobacterium halobium in Salzkristallen

Langsames Verdunsten des Wassers im Elektrofeld aus einer mit Bakterien gesättigten Lösung von Meerwasser führt zur Auskristallisation von Kochsalzkristallen. Sie enthalten lebendes Bakterienmaterial. Die Kristalle können getrocknet werden. In ein Elektrofeld von 750 V/cm eingebracht, können die Halobakterien im Kristall eingeschlossen über mehrere Jahre überleben. Sie vermehren sich sogar. Nach Auflösen des Kristalls in neuem Meerwasser sind die Bakterien voll lebensfähig. Ebenfalls voll lebensfähig bleiben Halobakterien, die im Salz eingeschlossen sind, wenn sie im Kühlschrank - ohne Feld - über längere Zeit ($\geq$ 1 Monat) bei -28° C aufbewahrt werden.

6.8. Auswirkungen eines statischen Elektrofeldes auf die Zusammensetzung und das Wachstum einer gemischten Bakterienpopulation

E. coli 205, K. pneumoniae 327, P. aeruginosa ATCC12055, S. aureus 10 B, S. pyogenes L-15 sowie Bacillus subtilis MX-1 werden unter aeroben Bedingungen über Nacht bei einer Temperatur von 37° C in „Brain Heart Infusion" (BHI)-Medium (Difco Manual, 10th Edition, Difco Laboratories Inc., Detroit, Mich., 1984, 160-162) angezogen. C. perfringens wird unter anaeroben Bedingungen ebenfalls über Nacht und auf BHI-Agar bei einer Temperatur von 37° C angezogen und die gewachsenen Kolonien in flüssigem BHI-Kulturmedium resuspendiert.

Die Übernachtkulturen werden in BHI-Medium verdünnt, bis eine gerade noch sichtbare Trübung der Suspension vorliegt. Je 1 ml der so hergestellten Suspensionen wird zu 13 ml einer vorgewärmten frischen BHI-Kulturlösung zugegeben, sodass ein Gesamtvolumen von 20 ml vorliegt.

Die Mischkulturen werden in 25 cm$^3$ Gewebekulturflaschen überführt, zwei der Flaschen in einem statischen Elektrofeld inkubiert, während zwei weitere als Kontrollen dienen. Die Inkubation erfolgt bei einer Temperatur von 37° C unter dreimaligem Schütteln der Kulturen pro Tag. Es werden täglich Proben entnommen und in einer geeigneten Verdünnung (die ca. 200 Kolonien pro Platte ergibt) auf Eosin-Methylenblau-Agar (zur Differenzierung von E. coli, K. pneumoniae und B. subtilis) [Difco Manual, Seite 307-308], auf Mannitol-Salz-Agar (S. aureus) ) [Difco Manual, Seite 558-560], Pseudomonas-Selektivagar (P. aeruginosa) [Difco Manual, Seite 709-711], Blutagar (2x, 1. aerob für S. pyogenes; 2. anaerob für C. perfringens) ausplattiert. Diese Medien erlauben die Bestimmung der Anzahl der einzelnen Komponenten der Mischkultur.

Ergebnis

Das statische Elektrofeld hat keinen Einfluss auf die Gesamtzahl der Organismen in der Mischkultur. Die Ergebnisse der Tabelle 6 zeigen aber, dass die Wechselwirkungen zwischen den verschiedenen Komponenten der Mischkultur durch das Feld beeinflusst werden und zu einer Zunahme einzelner Arten in der Gesamtpopulation führen. Dies geschieht in der Regel auf Kosten einer oder mehrerer anderer Spezies, die in der Mischkultur vorliegen. So ist beispielsweise der Anteil an P. aeruginosa 24 Stunden nach Versuchsbeginn unter Feldbedingungen leicht erhöht im Vergleich zu den Kontrollen. Diese Zunahme erfolgt auf Kosten von E. coli, das in den Kontrollkulturen in einem höheren Prozentsatz vertreten ist als in den Versuchskulturen.

Nach 5 Tagen Kulturdauer lässt sich eine dramatische Veränderung beobachten. In den Feldkulturen hat der Anteil an E. coli sehr stark zugenommen, während der Anteil an K. pneumoniae fast in gleichem Masse zurückgeht. Es liegen darüberhinaus gewisse Hinweise darauf vor, dass auch C. perfringens unter Feldbedingungen etwas besser wächst als in den Kontrollkulturen.

Tabelle 5:

Einfluss eines statischen Elektrofeldes auf die Zusammensetzung und das Wachstum einer gemischten Bakterienkultur

a  F1/F2 Unter Feldeinfluss kultivierte Proben; C1/C2 Kontrollkulturen
b  Gesamtzahl der einzelnen Spezies

| Zeit (d) | Kultur-flasche[a] | Gesamt log cfu/ml[b] | Zusammensetzung (%) | | | | | | |
|---|---|---|---|---|---|---|---|---|---|
| | | | E. coli | K. pneu-moniae | P. aeru-ginosa | S. aureus | S. pyoge-nes | B. subtilis | C. perfrin-gens |
| 0 | F1 | 7.05 | 17 | 26 | 5 | 1 | 1 | 42 | 8 |
| | F2 | 6.98 | 17 | 20 | 4 | 1 | n.d. | 59 | n.d. |
| | C1 | 6.97 | 14 | 28 | 2 | 1 | <1 | 43 | 12 |
| | C2 | 6.94 | 24 | 35 | 2 | 2 | n.d. | 37 | n.d. |
| 1 | F1 | 9.20 | 29 | 17 | 13 | <1 | 0 | 40 | 1 |
| | F2 | 9.21 | 28 | 14 | 9 | <1 | 0 | 48 | 1 |
| | C1 | 9.14 | 37 | 18 | 6 | <1 | 0 | 38 | 1 |
| | C2 | 9.21 | 40 | 19 | 5 | <1 | 0 | 35 | 1 |
| 2 | F1 | 9.08 | 15 | 15 | <1 | <1 | 0 | 66 | 4 |
| | F2 | 9.01 | 23 | 13 | <1 | <1 | 0 | 63 | <1 |
| | C1 | 9.18 | 29 | 9 | 1 | <1 | 0 | 60 | 1 |
| | C2 | 8.79 | 30 | 5 | 1 | <1 | 0 | 63 | <1 |
| 5 | F1 | 8.39 | 64 | 3 | 2 | 14 | 0 | 15 | 2 |
| | F2 | 8.35 | 51 | 5 | 2 | 17 | 0 | 11 | 2 |
| | C1 | 8.24 | 62 | 2 | 2 | 23 | 0 | 10 | 1 |
| | C2 | 8.16 | 70 | 3 | 3 | 12 | 0 | 11 | 2 |
| 6 | F1 | 8.17 | 66 | 4 | 8 | <1 | 0 | 19 | 3 |
| | F2 | 8.27 | 67 | 6 | 5 | <1 | 0 | 17 | 4 |
| | C1 | 8.18 | 3 | 74 | 4 | <1 | 0 | 15 | 3 |
| | C2 | 8.13 | 3 | 78 | 5 | <1 | 0 | 12 | 3 |

7. Beispiel: Dünnschichtchromatographie

a) Ein Chromatographietank für die Dünnschichtchromatographien (DC) wird auf den Aussenseiten mit Aluminiumplatten versehen, die mit einem Hochspannungsgenerator (FUG HCN 14-12500) leitend verbunden sind. Der Chromatographietank bildet so das Dielektrikum eines Kondensators. Die Platten werden mit einer Gleich-Spannung von 9'000 V aufgeladen, sodass eine elektrische Feldstärke von 2'670 V/cm zwischen den Platten herrscht. Der Tank wird in üblicher Weise mit dem Fliessmittel bestückt und die DC-Platten darin entwickelt. Man trägt auf Kieselgelplatten [(60 F 254 Muck) Nr. 5717 (5x20 cm)] in bekannter Weise 10 μl Testfarbstoffgemisch (lipophil) nach Stahl (Merck Art. Nr. 9353) auf und entwickelt sie in Tetrachlorkohlenstoff (Merck Art. Nr. 2209 Urasol) als Fliessmittel. Je eine solche Platte wird in der angegebenen Apparatur und parallel dazu in einem normalen gleich grossen Tank ohne Feld entwickelt. Die dabei entstehenden Chromatogramme werden verglichen.

| Farbstoff | $R_f$-Werte | |
|---|---|---|
| | ohne E-Feld | mit E-Feld |
| 1 | 0 | 0 |
| 2 | 0,066 | 0,016 |
| 3 | 0,133 | 0,166 |
| 4 | 0,433 | 0,216 |
| 5 | 0,483 | 0,233 |
| 6 | 0,866 | 0,483 |
| Steighöhe des Fliessmittels | 7,6 | 9,1 |

b) Man trägt auf Kieselgelplatten (60 F 254 Merck) Nr. 5717 (5x20 cm) in bekannter Weise 10 μl Testfarbstoffgemisch (hydrophil) (Merck Art. Nr. 9352) auf und entwickelt sie in Methanol/Wasser 1:1 als Fliessmittel. Je eine solche Platte wird in der angegebenen Apparatur und parallel dazu in einem normalen gleich grossen Tank entwickelt. Die dabei entstehenden Chromatogramme werden verglichen.

| Farbstoff | $R_f$-Werte | |
|---|---|---|
| | ohne E-Feld | mit E-Feld |
| 1 | 0,734 | 0,730 |
| 2 | 0,823 | 0,827 |
| 3 | 0,911 | 0,962 |
| Steighöhe des Fliessmittels | 5,3 | 8,4 |

In beiden Fällen wird die Diffusionsgeschwindigkeit des Fliessmittels erhöht. Die bedeutende Polarisierbarkeit des Methanols erscheint als Ursache für den besonders deutlichen Effekt beim polaren Fliessmittel.

**Patentansprüche**

1. Verfahren, welches basierend auf einer Modifikation chemisch/physikalischer Prozessabläufe in einfachen sowie in komplexen Systemen zu wünschenswerten und nützlichen Veränderungen von diesen Systemen inhärenten Eigenschaften führt und das durch die folgenden Verfahrensmassnahmen gekennzeichnet ist:

   a) Einbringen von einfachen oder komplexen Systemen, in denen die spezifischen chemisch/physikalischen Prozessabläufe stattfinden, welche mit Hilfe des erfindungsgemässen Verfahrens modifiziert werden sollen, in ein statisches Elektrofeld,

b) Einstellen der für die jeweils gewünschte Modifikation der im statischen Elektrofeld befindlichen Systeme geeigneten Feldparameter und

c) Belassen der besagten Systeme im statischen Elektrofeld für einen Zeitraum, der für das Auftreten und gegebenenfalls für die Erhaltung der gewünschten Modifikation notwendig ist.

2. Verfahren gemäss Anspruch 1, welches basierend auf einer Modifikation chemisch/physikalischer Prozessabläufe in einfachen sowie in komplexen biologischen Systemen zu wünschenswerten und nützlichen Veränderungen von diesen Systemen inhärenten Eigenschaften führt und das durch die folgenden Verfahrensmassnahmen gekennzeichnet ist:

a) Einbringen von biologischem Material in ein statisches Elektrofeld,

b) Einstellen der für die jeweils gewünschte Modifikation geeigneten Feldparameter und

c) Belassen des biologischen Materials im statischen Elektrofeld für einen Zeitraum, der für das Auftreten und gegebenenfalls für die Erhaltung der gewünschten Modifikation notwendig ist.

3. Verfahren gemäss Anspruch 1, dadurch gekennzeichnet, dass das statische Elektrofeld zwischen den Platten eines Kondensators von beliebiger Form aufgebaut wird.

4. Verfahren gemäss Anspruch 1, dadurch gekennzeichnet, dass die Feldstärkewerte des statischen Elektrofeldes zwischen 1 V/cm und 10'000 V/cm betragen.

5. Verfahren gemäss Anspruch 2, dadurch gekennzeichnet, dass es sich bei dem zu modifizierenden biologischen Material um einen intakten lebenden Organismus handelt.

6. Verfahren gemäss Anspruch 5, dadurch gekennzeichnet, dass es sich um einen intakten lebenden Organismus, ausgewählt aus der Gruppe bestehend aus Mikroorganismen, Pilzen, Pflanzen, wirbellosen Tieren und Wirbeltieren aus den Klassen der Amphibien, Reptilien, Vögel und Säuger, handelt.

7. Verfahren gemäss Anspruch 2, dadurch gekennzeichnet, dass man biologisches Material verwendet, das eine hohe Teilungsaktivität aufweist und/oder noch wenig differenziert ist, ausgewählt aus der Gruppe bestehend aus teilungsaktiven Zellen, Geschlechtszellen während des Befruchtungsvorgangs, Keimzellen, embryonalen Zellen und Geweben, Embryonen, Zygoten, Keimlingen sowie meristematischen Zellen und Geweben.

8. Verfahren gemäss Anspruch 6 oder 7 zur Steigerung des Wachstums der aus dem behandelten Samen-/Sporenmaterial erhaltenen Pflanzen.

9. Verfahren zur Stabilisierung der durch einen Mangelstress induzierten Produktionsvarianten eines zur Produktion von Sekundärmetaboliten befähigten Mikroorganismus, dadurch gekennzeichnet, dass man

a) besagten Mikroorganismus in ein geeignetes Kulturmedium einbringt und

b) diesen im Einflussbereich eines statischen Elektrofeldes kultiviert,

c) die Kultivierung solange fortsetzt, bis sich die Produktionsform ausbildet und stabilisiert hat und

d) den produzierten Sekundärmetaboliten aus dem Medium oder dem Zellmaterial isoliert.

10. Verfahren gemäss Anspruch 9, dadurch gekennzeichnet, dass es sich um ein Rhodopsin- und/oder $\beta$-Carotin-produzierendes Halobakterium handelt.

MG-Bereich                                          Kathode

94'000 —

68'000 —

43'000 —

30'000 —

21'000 —

14'300 —

      1      2      3          1      2      3

       (GLOBULINE)              (ALBUMINE)

Abbildung 1:   SDS-PAGE   RAINERI Proteine

              [ 1  Kontrolle ; 2 Körner aus dem Haupttrieb;
                3  Körner aus dem Seitentrieb ]

EP 0 791 651 A1

**EUROPÄISCHER RECHERCHENBERICHT**

Nummer der Anmeldung

EP 97 10 0938

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE 37 35 702 A (ATHENSTAEDT HERBERT DR DR) 16.März 1989 * das ganze Dokument * | 1-7,9 | C12N13/00 C12P23/00 C12P1/04 A01G7/04 //(C12P1/04, C12R1:01), (C12P23/00, C12R1:01) |
| X | GB 1 090 011 A (TECHNOIMPEX MAGYAR GEPIPARI KULKEREKEDELMI VALLALAT) * Seite 1, Zeile 12 - Zeile 65; Ansprüche; Abbildungen * * Seite 2, Zeile 33; Tabelle * | 1-8 | |
| X Y | US 4 154 668 A (ZIMMERMANN U. ET AL.) 15.Mai 1979 * Spalte 2, Zeile 35 - Zeile 49; Ansprüche; Abbildungen * * Spalte 1 * * Spalte 4 * | 1-6 9 | |
| X Y | US 4 292 408 A (ZIMMERMANN ULRICH ET AL) 29.September 1981 * Ansprüche; Beispiele * | 1-6 9 | |
| X | WO 86 03780 A (TECHNICLONE RESEARCH PARTNERS) 3.Juli 1986 * Ansprüche * | 1-6 | RECHERCHIERTE SACHGEBIETE (Int.Cl.6) C12N C12P |
| X | DE 10 57 812 B (FRITZ HAHN) 21.Mai 1959 * Seite 1; Ansprüche * | 1-6 | |
| X | US 2 831 804 A (J.COLLOPY) * Ansprüche; Beispiele * | 1-6 | |
| X | FR 1 451 661 A (G. DEFAUT) 5.Dezember 1966 * Seite 1 - Seite 9; Abbildungen * | 1-9 | |
| X | DE 22 13 905 A (RIESS REINHOLD) 4.Oktober 1973 * das ganze Dokument * | 1-8 | |

-/--

Der vorliegende Recherchenbericht wurde für alle Patentansprüche erstellt

| Recherchenort | Abschlußdatum der Recherche | Prüfer |
|---|---|---|
| DEN HAAG | 28.Mai 1997 | Coucke, A |

EPO FORM 1503 03.82 (P04C03)

Europäisches
Patentamt

**EUROPÄISCHER RECHERCHENBERICHT**

Nummer der Anmeldung

EP 97 10 0938

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | WO 86 03372 A (NOGLER & DAUM ELTAC) 19.Juni 1986 * Ansprüche * --- | 1-6,8 | |
| X | FR 2 178 647 A (SENSORS INC) 9.November 1973 * Ansprüche; Beispiele * --- | 1-6,8 | |
| X | CH 495 772 A (CHEMOLIMPEX) 15.September 1970 * Ansprüche; Beispiele * --- | 1-8 | |
| X | GB 2 100 112 A (TOPLINE MEHESZ DEVELOPMENTS LT) 22.Dezember 1982 * Ansprüche; Beispiele * --- | 1-8 | |
| X | PATENT ABSTRACTS OF JAPAN vol. 005, no. 008 (C-039), 20.Januar 1981 & JP 55 135586 A (KUBOZONO YOSHIAKI), 22.Oktober 1980, * Zusammenfassung * --- | 1-8 | RECHERCHIERTE SACHGEBIETE (Int.Cl.6) |
| X | US 5 458 752 A (H.M. LIZAMA ET AL.) 17.Oktober 1995 * Ansprüche; Abbildungen * ----- | 1-6,9 | |

Der vorliegende Recherchenbericht wurde für alle Patentansprüche erstellt

| Recherchenort | Abschlußdatum der Recherche | Prüfer |
|---|---|---|
| DEN HAAG | 28.Mai 1997 | Coucke, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
......................................................
& : Mitglied der gleichen Patentfamilie, übereinstimmendes
    Dokument

EPO FORM 1503 03.82 (P04C03)